# EUROPÄISCHE PATENTSCHRIFT

(11) **EP 3 449 741 B1**
(45) Veröffentlichungstag und Bekanntmachung des Hinweises auf die Patenterteilung: **14.04.2021**
(21) Anmeldenummer: 18000701.5
(22) Anmeldetag: 29.08.2018
(51) Int. Cl.: A61F 13/42, G01N 33/52, G01N 21/78

(54) **VORRICHTUNG ZUR EINWANDFREIEN BESTIMMUNG DER NUTZUNG EINES BEKLEIDUNGSSTÜCKS**
DEVICE FOR THE CORRECT IDENTIFICATION OF THE USE OF AN ARTICLE OF CLOTHING
DISPOSITIF DESTINÉ À LA DÉTERMINATION CORRECTE DE L'UTILISATION D'UN VÊTEMENT

(30) Priorität: 30.08.2017 DE 102017008178
(43) Veröffentlichungstag der Anmeldung: 06.03.2019
(73) Patentinhaber: Schmitz-Brackmann, Melanie, 46119 Oberhausen (DE)
(72) Erfinder: Schmitz-Brackmann, Melanie, 46119 Oberhausen (DE)
(74) Vertreter: Gehrke, Peter P.

(56) Entgegenhaltungen:
- EP-A2- 0 403 876
- DE-A1-102011 003 517
- US-A- 2 156 880
- US-A1- 2012 196 375
- US-A1- 2015 265 475
- US-A1- 2015 339 726

## Beschreibung

Die Erfindung betrifft die Verwendung einer zuverlässigen Vorrichtung zur einwandfreien Bestimmung der durch einen Benutzer erfolgten Nutzung eines Bekleidungsstücks, insbesondere eines Ober- und Unterbekleidungsstückes, mit einer Einlage zur Anordnung auf einem dem Benutzer zugewandten innenseitigen Abschnitt desselben, wie Schritt- oder Achselbereich, und zum Auffangen menschlicher Ausscheidungen, insbesondere Urin, Schweiß, Blut, und eine Vorrichtung, die die Nutzung des Bekleidungsstücks erfasst.

Im Stand der Technik ist bei dem Versand von neuen Bekleidungsstücken und Schuhen das sich aufdrängende Problem bekannt, dass die lediglich zum Anprobieren zugesandten Waren für eine bestimmte Zeitdauer lang widerrechtlich ohne Zustimmung des Versenders genutzt werden können, so dass die Bekleidungsstücke beschädigt oder zumindest abgetragen sind, welche Umstände sich durch die Anhaftung von Schweiß, Nässe, Verunreinigungen, usw. zeigen. Insbesondere weist die zurückgesandte Unterwäsche im Fall der Dauernutzung unerfreuliche Rückstände von Ausscheidungen des Benutzers auf, die zu der Unverkäuflichkeit der Unterwäsche beitragen.

Zudem zeigt sich, dass auch versandte Schuhe infolge der Nutzung -statt lediglich des Anprobierens- einen Abrieb im Schuhsohlenbereich, zumindest aber die Aufnahme von Gerüchen, aufweisen, so dass die an den Versender zurückgesandten Schuhe nicht als neuwertige Waren verkaufbar sind und teilweise entsorgt oder als gebrauchte Schuhe mit Verringerung des erhofften Werterlöses weiterverwertet werden müssen.

Der hierdurch entstehende Schaden ist derart hoch, dass von Seiten der Versender nach Abhilfe bereits seit langem gesucht wird, die über das Anprobieren hinausgehende Nutzung der Bekleidungsstücke und Schuhe zweifelsfrei, eindeutig und ohne Weiteres nachweisbar machen.

Da in zunehmender Weise mit den o.g. Waren weniger über den Einzelhandel, vielmehr über den online-Handel beworben wird, verstärkt sich das Problem der unerlaubten Nutzung solcher Art von Waren.

Die Druckschriften US 2156880 und EP 0403876 zeigen eine Vorrichtung und eine Verwendung einer Vorrichtung gemäß der Präambel der unabhängigen Ansprüche.

Die DE 29 22 470 betrifft zwar einen Sanitärartikel oder ein Sanitärtuch, die Indikatoren aufweisen, um krankhafte Zustände des Benutzers derselben durch die Abgabe von Körpersekreten des Benutzers festzustellen. Der Sanitärartikel kann beispielsweise Unterhosen oder in Bekleidung aufnehmende absorbierende Polster sein, die mit einem Reagenz getränkt sind, welches in Form eines Papierstreifens auf dem Artikel anliegt. Durch die Absonderung von Körpersekreten reagieren die Reagenzien des Papierstreifens auf beispielsweise Urin, Schweiß, Kot. Der absorbierende Träger kann sein Papier, Gewebe, Bänder oder dergleichen.

Auch die DE 36 19 853 ist auf ein Verfahren zum Färben von Schlauchwaren mit Reaktivfarbstoffen, welches Verfahren die besondere Behandlung der Schlauchware enthaltende Zellulose als Fasern umfasst und die besondere Behandlung von Farbstoffen zur Erhöhung deren Affinität zu Fasern beschreibt; jedoch erkennt dieser Stand der Technik nicht das Erfordernis der Bereitstellung einer Vorrichtung zur einwandfreien Bestimmung der Nutzungsdauer eines Bekleidungsstücks und Schuhwerks.

Auch wenn den in Rede stehenden Waren infolge der unerlaubten Nutzung zumindest Schweißgeruch anhaftet, der für die sensorische Prüfung auffällig ist, ist die sensorische Prüfung bereits wegen der individuell unterschiedlichen Ausprägung des Empfindens des Geruchs, z.B. durch seine Stärke, seine Zusammensetzung, unzureichend, da der Geruchssinn des Menschen in Bezug auf seine Wahrnehmung und Empfindung wegen der verschieden starken Ausbildung von Geruchszellen in einem Geruchsorgan keine einheitliche Geruchserfassung und -bestimmung zulässt.

In der Druckschrift DE 10128086 A1 ist ein markiertes Inkontinenzmittel offenbart, welches eine Markierungssubstanz aufweist, das in der Gegenwart von Wasser einen Farbumschlag bewirkt, sodass das herkömmliche Inkontinenzmittel die Aufnahme von Wasser in dieses anzeigt. Die Markierungssubstanz kann beispielsweise metalloide oder metallische Inhaltsstoffe enthalten, die an Sauerstoff gebunden vorliegen und in Gegenwart von Wasser das herkömmliche Inkontinenzmittel einen Farbkomplexbildner erzeugen. Der Farbkomplexbildner ist zwar in der Lage, das Eintreten von Wasser in das herkömmliche Inkontinenzmittel qualitativ anzuzeigen, aber die Dauer des Eindringens von Wasser in das das herkömmliche Inkontinenzmittel mittels eines quantitativen Merkmals wird nicht infolge des Farbumschlags vermittelt.

Die qualitative Anzeige des Eindringens von Wasser in das herkömmliche Inkontinenzmittel reicht bereits aus, um das Auswechseln des vernässten herkömmlichen Inkontinenzmittels vorzubereiten. Das Ausmaß der Vernässung des Inkontinenzmittels verbleibt unbeachtlich, so dass auch keine Maßnahmen vorgeschlagen werden, die die Erfassung des Ausmaßes der Vernässung mittels zu messender Größen als Messgrößen bestimmen; das Problem der quantitativen Bestimmung der mengenmässigen Vernässug ist aber offensichtlich lösbar. Daher gibt die Druckschrift DE 10128086 A1 auch keinen Hinweis, auf welche Weise ein markiertes Inkontinenzmittel darstellbar wäre, das erst den Eintritt der Vernässung des herkömmlichen Inkontinenzmittels nach einer vorbestimmten längeren Zeitdauer vermittelt.

Das herkömmliche Inkontinenzmittel zeichnet sich durch eine rasche Farbumschlagreaktion aus, insbesondere, wenn die Komplexbildung mit schwer löslichem dreiwertigem Eisen enthaltenden Oxiden in der Gegenwart von Reduktionsmitteln durchgeführt wird. Infolge des Wasserkontaktes mit der Markierungssubstanz während des Einsatzes des herkömmlichen Inkontinenzmittels kommt es zur Reaktion des Komplexbildners mit dem Metall, Oxid und/oder Mischoxid und infolge des Reduktionsmittels tritt der Farbumschlag ein. Als Reduktionsmittel können ungiftige Verbindungen, wie Ascorbinsäure und deren Derivate, insbesondere das Kalziumsalz der Ascorbinsäure und Esther und Anhydride der Ascorbinsäure mit Alkoholen und Carbonsäuren als Reduktionsmittelkomponenten in der Markierungssubstanz zum Einsatz in dem herkömmlichen Inkontinenzmittel gelangen.

Eine weitere Hilfestellung durch die Druckschrift erfährt der Fachmann nicht, auf welche Weise die Nutzung oder das Anprobieren eines Bekleidungsstücks oder Schuhe zeitmäßig überprüft werden kann, um einen eindeutigen, zweifelsfrei ohne weiteres ersichtlichen Nachweis der Nutzung oder des Anprobierens desselben bereitzuhalten.

Auch in der G 851 13 47.6 wird eine herkömmliche Windel beschrieben, die ein Feuchtigkeitsabsorbierendes Material enthält, in den dem Körper am nächsten liegenden Lagen im Bereich des Unterleibes des Benutzers lochförmige Ausschnitte angebracht sind, die in der Windel Mulden bilden, wobei zudem mindestens eine Trägerlage vorgesehen ist, die keine lochförmigen Ausschnitte aufweist. Auch die weiteren Vorschläge des Zuschnitts der Windel, um diese möglichst den anatomischen Verhältnissen des Benutzers anzupassen, und die Bereitstellung einer Vliesummantelung führen nicht weiter, eine Vorrichtung bereitzuhalten, die die Nutzungsdauer eines Bekleidungsstücks oder Schuhwerks nachweisen kann. Im Sinne der Erfindung werden unter Benutzer auch Benutzerrinnen verstanden.

Die Druckschrift gibt lediglich dem Benutzer den Rat, den Bereich der Windel dergestalt auszugestalten, dass ein größtmöglicher Schutz vor Windeldermatitis geboten wird. Auch wenn die herkömmliche Windel den Unterleib des Benutzers vor austretenden Exsudaten, wie Kot, Urin, schützt, zeigt der Feuchtigkeitsindikator, der in dem über eine Leiste verlaufenden Endbereich einer Trägerlage eingebracht ist, lediglich einmalig, also qualitativ, -dem Alles oder Nichts Prinzip folgend- die Befeuchtung der Windel an, hingegen wird keine Quantifizierung des Benutzungsnachweises der Windel beispielsweise nach mehrmaliger Benutzung oder bestimmter Benutzungsdauer verwirklicht. Daher hilft auch die Offenbarung dieser Druckschrift nicht weiter, Bekleidungsstücke und Schuhwerk und deren mehrmaligen Nutzung zuverlässig zu überwachen.

Des Weiteren beschreiten die in der DE - OS 29 22 470 beschriebenen Sanitärartikel, wie Windeln, absorbierende Polster, Unterhosen, usw., den Weg, den Benutzer zu informieren, dass bestimmte Substanzen in den Exsudaten, wie Sekrete, Urin, Blut, Kot, die von dem Benutzer ausgeschieden bzw. abgegeben werden, Zeichen pathologischer Zuständen des Benutzers sein können. Ein solcher Farbwechsel soll nach Lehre der Druckschrift dadurch erreicht werden, dass eine Zone in dem herkömmlichen Sanitärartikel bereitgestellt wird, die ein Farbreagenz aufweist, beispielsweise durch Aufsprühen, Aufschmieren, Tränken und dergleichen, die in der Lage ist, durch einen qualitativen Farbumschlag die Anwesenheit bestimmter Substanzen in dem Sekret anzuzeigen. So wird in der Druckschrift die Verwendung von Methylorange (Helianthen) vorgeschlagen, ein Farbreagenz, welches auf den Säuregrad des Urins durch Farbumschlag reagiert.

Auch in Kenntnis dieser Druckschrift wird die Bereitstellung einer Vorrichtung nicht unterstützt, um die Nutzung und deren Dauer der Nutzung des Bekleidungsstücks oder Schuhwerks nachzuweisen, denn Aufgabe einer solchen bereitzustellenden Vorrichtung soll es nicht sein, bestimmte, ggf. auf Krankheiten diagnostizierende Reaktionen anzuzeigen sondern, vielmehr auf einfache Weise die Nutzungsdauer des Bekleidungsstücks darzulegen, sodass der Nachweis erwünscht ist, die Dauer der Nutzung des Bekleidungsstück quantitativ anzuzeigen.

Überdies soll die Einlage in der Lage sein, preiswert herstellbar und für den ungeübten Benutzer leicht handhabbar zu sein, ohne das Bekleidungsstück infolge Anbringens oder Ansetzens der Vorrichtung gegen das Bekleidungsstück zu beschädigen, und geschwiege denn, das Tragekomfortgefühl bei Anprobieren des Bekleidungsstücks einzuschränken.

Schließlich wird in der US 2013/0289508 eine herkömmliche Windel dargestellt mit einem Reaktionsmittel zum Nachweis der Benutzung derselben, die ebenso wie die vorhergehenden Druckschriften das einmalige Einbringen von Sekreten des Benutzers nur qualitativ nachzuweisen vermag, aber nicht zur quantitativen Bestimmung der Dauer der Benutzung der Windel anwendbar ist; das der herkömmlichen Lehre zugrunde liegende Prinzip dient der Nutzung der Windel bis Erreichen eines vorbestimmten Feuchtigkeitszustandes, unterscheidet aber nicht die kurzfristig andauernde Nutzung von der langfristigen Nutzung.

Infolge des technischen Erfolgs der herkömmliche Lehre und deren Nachteile, die in den vorgenannten Druckschriften beschrieben werden, die weder der Erfindung zugrunde liegende Aufgabe beschreiben, noch Hilfestellung zur Lösung derselben geben, sind Anstrengungen zu unternehmen, um eine Vorrichtung bereitzuhalten, die die mehrmalige Nutzungen und die dauernde Nutzung von der einmaligen kurzandauernden Nutzung unterscheidet.

Es reicht folglich nicht aus, das Bekleidungsstück oder einen Teil des Bekleidungsstücks mit einem Reaktionsmittel, welches als Farbreagenz in Kontakt mit Exsudaten des Benutzers zu reagieren vermag zur Erzeugung eines Farbumschlags, zu vermengen, ggf. sogar, wie in dem Stand der Technik vorgeschlagen, Teile desselben an Körperformen des Benutzers anzupassen.

Die Einmaligkeit der Erzeugung eines visuellen Signals als Farbumschlag führt nach Lehre der Druckschriften zwar den Benutzer zu der Erkenntnis, es läge die Benutzung des Bekleidungsstücks vor, die Erkenntnis führt aber nicht weiter, denn es ist nicht wesentlich die Nutzung eines Bekleidungsstücks nachzuweisen, sondern vielmehr die Nutzungsdauer des Bekleidungsstücks eindeutig zu bestimmen.

Daher ist es auch Aufgabe der Erfindung, einen solchen Lösungsweg einzuschlagen, der von den herkömmlichen Lehren, die auf die Einmaligkeit der Benutzung, wenn überhaupt, des Erzeugnisses abstellen, wegführt und dem Benutzer die Möglichkeit bietet, abgesehen von dem Nachweis der Einmaligkeit der Nutzung als qualitativer Nachweis, auch den Nachweis der dauerhaften Nutzung, die die Summe der Mehrmaligkeit der Nutzungen einschließen kann, als quantitativer Nachweis möglich zu machen.

Daher ist es Aufgabe der Erfindung, die Nutzung der Bekleidungsstücke und Schuhe eindeutig zweifelsfrei ohne Weiteres ersichtlich nachzuweisen.

Da der online-Handel stark anwächst, gleichwohl dieser durch die mit dem Anprobieren der Bekleidungsstücke und Schuhe häufig einhergehende Abnutzung derselben in seinem Wachstum zumindest behindert wird, besteht ein hohes Bedürfnis im Handel, die unerlaubte Abnutzung der Bekleidungsstücke und Schuhe schon durch das Bekanntsein von zweifelsfreien, eindeutigen Nachweisverfahren zu unterbinden.

Im Stand der Technik sind keine Verfahren bekannt, die Nutzung der Bekleidungsstücke und Schuhen zu erfassen, so dass hierdurch ein hoher volkswirtschaftlicher Schaden entsteht, der nur unzureichend erfasst wird.

Aufgabe der Erfindung ist es, eine Vorrichtung und ein Verfahren bereitzustellen, die eindeutig nicht nur die Nutzung der Bekleidungsstücke und Schuhe, sondern auch die Dauer der Nutzung nachzuweisen vermag.

Ebenso sollen die Vorrichtung und das Verfahren bereits das einmalige Anprobieren der Bekleidungsstücke und Schuhe erfassen.

Ebenfalls die Vorrichtung leicht tragbar sein und nicht das Tragegefühl des Benutzers beeinträchtigen.

Die fehlende Beeinträchtigung ist wesentlich zum Beispiel bei Unterwäsche, da bei Unterwäsche bereits geringe, die Verformbarkeit des Textils einschränkende Strukturen in derselben das Wohlbefinden des Benutzers beeinflusst und dieses die Ablehnung des Erwerbs durch den Benutzer erfährt.

Daher sollen unangenehme oder negative Einflüsse der Vorrichtung und des Verfahrens vermieden werden.

Hinzukommend soll die Vorrichtung preisgünstig in der Herstellung und in seiner Anbringung in dem Bekleidungsstück und Schuhen einfach sein, ohne dass die Gefahr besteht, dass das Entfernen der Vorrichtung aus dem Bekleidungsstück und dem Schuh nicht ersichtlich ist.

Hinzutretend soll die an das Bekleidungsstück und Schuhwerk gekoppelte Vorrichtung ohne Einschränkung mehrmalig in ein und demselben Bekleidungsstück bzw. Schuhwerk benutzt werden können.

Überdies soll die Vorrichtung nach dem Erwerb der Ware und infolgedessen der erlaubten Nutzung derselben für den Benutzer leicht entfernbar sein.

Die Aufgaben werden gelöst durch die Hauptansprüche, die Unteransprüche betreffen bevorzugte Ausgestaltungen und Weiterentwicklungen der Erfindung.

Die Erfindung bezieht sich eine Verwendung der Vorrichtung zur Bestimmung der durch einen Benutzer erfolgten Nutzung eines Bekleidungsstücks, insbesondere eines Ober- und Unterbekleidungsstückes, mit einer Einlage zur Anordnung auf einem dem Benutzer zugewandten innenseitigen Abschnitt, wie Innenseite, desselben, z.B. des Schritt- oder Achselbereichs, und zum Aufnahme oder -fangen menschlicher Ausscheidungen, auch Exsudate genannt, insbesondere Urin, Schweiß, Kot, Blut, welche dadurch gekennzeichnet ist, dass
die Einlage Indikatormittel umfasst, wobei die Indikatormittel mindestens eine Indikator-Verbindung aufweist,
welche infolge ihrer Reaktion mit für menschliche Ausscheidungen charakteristischen Substanzen einen Farbumschlag zeigt,
z.B. die Einlage als ein textiles und/oder papierenes Flächengebilde ausgestaltet ist.

Ein weiterer Gegenstand der Erfindung ist gerichtet auf die Vorrichtung zur Bestimmung der durch einen Benutzer erfolgten Nutzung eines Bekleidungsstücks, insbesondere eines Ober- und Unterbekleidungsstückes, mit einer Einlage zur Anordnung auf einem dem Benutzer zugewandten Innenseite desselben, wie von Schritt- oder Achselbereich, und zum Aufnahme oder -fangen menschlicher Exsudate, welche dadurch gekennzeichnet ist, dass
die Einlage Indikatormittel umfasst, wobei die Indikatormittel mindestens eine Indikator-Verbindung aufweist, welche infolge ihrer Reaktion mit für das Exsudat charakteristischen Substanzen einen Farbumschlag zeigt,
die Einlage als ein textiles und/oder papierenes Flächengebilde ausgestaltet ist.

Die Erfindung kann auch sich beziehen auf eine Verwendung der Vorrichtung zur Bestimmung der durch einen Benutzer erfolgten Nutzung eines Bekleidungsstücks, insbesondere eines Ober- und Unterbekleidungsstückes, mit einer Einlage zur Anordnung auf einem dem Benutzer zugewandten innenseitigen Abschnitt, wie Innenseite, desselben, wie Schritt- oder Achselbereich, und zum Auffangen menschlicher Exsudate, insbesondere Urin, Schweiß, Blut, welche dadurch gekennzeichnet ist, dass
die Einlage Indikatormittel umfasst, wobei die Indikatormittel mindestens eine Indikator-Verbindung aufweisen, welche infolge ihrer Reaktion mit für Exsudate charakteristischen Substanzen einen Farbumschlag zeigt,
eine die Dauer der Nutzung des Bekleidungsstücks über mindestens eine für die Dauer charakteristische Messgröße erfassende elektronische Sensoreinrichtung zur Bestimmung der Abnutzung des Bekleidungsstücks umfasst.

Ein zusätzlicher Gegenstand der Erfindung ist gerichtet auf die Einlage und /oder Verwendung derselben zur Bestimmung der durch einen Benutzer erfolgten Nutzung eines Bekleidungsstücks, insbesondere eines Ober- und Unterbekleidungsstückes, mit einer Einlage zur Anordnung auf einem dem Benutzer zugewandten Innenseite desselben, wie von Schritt- oder Achselbereich, und zum Auffangen menschlicher Exsudate, welche dadurch gekennzeichnet ist, dass die Einlage Indikatormittel, wobei die Indikatormittel mindestens eine Indikator-Verbindung aufweisen, welche infolge ihrer Reaktion mit für Exsudate charakteristischen Substanzen einen Farbumschlag zeigt, die Einlage als ein textiles und/oder papierenes Flächengebilde ausgestaltet ist.

Die Erfindung umfasst die Vorrichtung zur Bestimmung der durch einen Benutzer erfolgten Nutzung eines Bekleidungsstücks, insbesondere eines Ober- und Unterbekleidungsstückes, mit einer Einlage zur Anordnung auf einem dem Benutzer zugewandten innenseitigen Abschnitt desselben, wie Schritt- oder Achselbereich, und zum Auffangen menschlicher Exsudate, insbesondere Urin, Stuhl, Schweiß, Blut, welche dadurch gekennzeichnet ist, dass die Einlage Indikatormittel umfasst, wobei die Indikatormittel mindestens eine Indikator-Verbindung aufweisen, welche infolge ihrer Reaktion mit für Exsudate charakteristischen Substanzen einen Farbumschlag zeigt, und eine die Dauer der Nutzung des Bekleidungsstücks über mindestens eine für die Dauer charakteristische Messgröße erfassende elektronische Sensoreinrichtung zur Bestimmung der Abnutzung des Bekleidungsstücks umfasst.

Die erfindungsgemäße Vorrichtung umfasst eine Einlage. Unter Einlage wird im Sinne der Erfindung auch ein flächiges, vorzugsweise längliches, z.B. ein viereckiges, Gebilde, z.B. mit abgerundeten Ecken, verstanden. Im Falle der Ausgestaltung als viereckiges längliches flächenhaftes Gebildes kann dieses beispielsweise konkav oder konvex -je nach Beschaffenheit des zu verwendenden Bekleidungsstücks geformt sein. Die Einlage kann einschichtig mit einer Deckschicht oder mehrschichtig, wie zweischichtig mit der Deckschicht und einer Trägerschicht, oder dreischichtig mit der Deckschicht, der Trägerschicht, einer auf der Deckschicht angeordneten Außen- oder Oberschicht oder auf der Unterseite der Trägerschicht angeordneten Sperrschicht oder vierschichtig zusammen mit den vorgenannten Schichten ausgebildet sein. So kann des Weiteren die Einlage als ein mehrschichtiges Formstück oder Laminat z.B. Schichtungsstoff, mit der Deckschicht und der Trägerschicht ausgestaltet sein, wobei die Schichten mindestens abschnittsweise miteinander verbunden oder aneinander gekoppelt sein können. Die Einlage kann zusätzlich an der Unterseite der Deckschicht und/oder an der Unterseite der Trägerschicht mit einer Sperrschicht aus einem flüssigkeitsdichten, wie wasserdichten, Material versehen sein, um das Austreten der mit dem Exsudat reagierten Indikator-Verbindung und Ausbreiten des Farbumschlags aus der Deckschicht und/oder der Trägerschicht in den Stoff des Bekleidungsstücks, z.B. in den Stoff des Schrittbereichs, zu verhindern und ggf. Verfärbungen des Stoffs, Benetzung des Stoffs mit der Indikator-Verbindung, dem als den Farbumschlag erzeugenden Reaktionsprodukt als Reaktion des Exsudates oder Bestandteilen desselben mit der Indikator-Verbindung zu vermeiden, so dass der Bekleidungsstück vorteilhafterweise unbeschädigt verbleibt. Die Sperrschicht kann eine herkömmliche wasserdichte und wasserdampfdichte Folie sein, die üblicherweise Kautschuk und/oder Kunststoffe enthält. Hinzutretend kann die Trägerschicht mit dem flüssigkeitsdichten, wasserdichten, Material, die üblicherweise eine Kautschuk-Verbindung und/oder Kunststoffe sein kann, versehen, insbesondere getränkt, sein.

Die Einlage ist als ein textiles oder papierenes Flächengebilde vorteilhafterweise ausgestaltet, welches als textiles Gewebe, Gewirke, Gestricke, Gelege, Verbundstoff, Vliesstoff, Wirk- oder Strickware ausgestaltet sein kann.

Im Sinne der Erfindung kann der Fachmann unter einem textilen Gewebe ein sich aus kreuzenden Fäden umfassender Stoff auch verstehen, welcher z.B. im rechten Winkel kreuzende Fäden aufweist, die als Kette und Schuss oder Kettfaden und Schussfaden benannt werden. Der obere Bereich der Einlage, der in Kopplungsstellung der Einlage in dem Bekleidungsstück dem Körper des Benutzers zugewandt ist, ist flüssigkeitsaufsaugend ausgestaltet, so dass der Bereich die Ausscheidung aufnimmt. Unter Kopplungsstellung wird im Sinne der Erfindung auch der vernähte Zustand der Einlage mit dem Bekleidungsstück verstanden.

Die Vorrichtung und die Verwendung einer Vorrichtung zur Bestimmung der durch einen Benutzer erfolgten Nutzung eines Bekleidungsstücks weist der obere Bereich der Einlage mehrere Abschnitte auf, die textile Flächengebilde mit unterschiedlicher Zusammensetzung und mindestens eine Indikator-Verbindung enthalten.

Der obere Bereich der Einlage weißt mehrere Abschnitte auf, die textile Flächengebilde mit unterschiedlicher Zusammensetzung und mindestens eine Indikator-Verbindung enthalten, wobei der erste Abschnitt einen Vliesstoff mit verwirbelten Filamenten und mindestens ein zweiter Abschnitt als textiles Flächengebilde einen Vliesstoff mit verwirbelten und glatten Filamenten umfassen, vorzugsweise kann ein dritter Abschnitt einen Vliesstoff mit glatten Filamenten oder Gewebe mit denselben umfassen. Durch die unterschiedlichen Zusammensetzungen der textilen Flächengebilde, z.B. der Vliesstoffe, der Abschnitte werden absorbierende Materialien dem Exsudat und dessen Bestandteilen ausgesetzt, welche vorteilhafterweise unterschiedlich schnell, und damit zeitabhängig. von diesen Vliesstoffen absorbiert und mit der Indikator-Verbindung, mit welchen die Vliesstoffe versehen sind, unter Bildung von Farbumschlägen reagieren können. Der Farbumschlag kann infolge Reaktion mit Exsudat unverändert verbleiben als qualitativer Nachweis, unabhängig von z.B. dem Ausmaß der Dauer der Nutzung, oder sich verstärken in Abhängigkeit von der Zeitdauer der Nutzung als quantitativer Nachweis.

Die Einlage oder erfindungsgemäße Vorrichtung mit derselben kann einen oberen Bereich, einen unteren Bereich, ggf. zwischen dem oberen Bereich und dem unteren Bereich angeordneten mittleren Bereich oder mindestens einen mittleren Bereich umfassen. Im Sinne der Erfindung wird unter Bereich auch eine Schicht oder eine Lage verstanden. So kann die Einlage als oberen Bereich eine Deckschicht aufweisen, die mindesten zwei Abschnitte aufweist bzw. in diese unterteilt ist. Die Abschnitte des oberen Bereichs oder der Deckschicht der Einlage können sich durch Zusammensetzung oder den Gehalt an textilen Flächengebilden voneinander unterscheiden. So kann in einer Ausgestaltung der erfindungsgemäßen Verwendung der Vorrichtung der erste Abschnitt der Deckschicht als textiles Flächengebilde einen Vliesstoff aufweisen, der Filamente umfasst, die durch Texturierung verwirbelt ausgebildet sein können. Es zeigt sich, dass Filamente infolge der herkömmlichen Texturierung eine besondere Textur, wie Gestalt, durch die maschinelle Behandlung von thermoplastischen Chemiefasern als Filamente, insbesondere Synthesefasern, in Form von Filamenten, Filamentgarnen erfahren.

Die texturierten Filamente können sich in Abhängigkeit von der durch die Texturierung besonderen Gestaltung der Filamente durch erhöhte mechanische Beanspruchbarkeit, ein größeres Volumen und damit einhergehend durch Bauschigkeit auszeichnen; die mit den texturierten Filamenten hergestellten textilen Flächengebilde, weisen hierdurch eine erhöhte Feuchtigkeitsaufnahme und Wasseraufnahme auszeichnen. So können durch die Texturierung aus z.B. glatten Filamenten verwirbelte Filamente, gestauchte Filamente erzeugt werden. Die Herstellung von textilen Flächengebilden mit verwirbelten Filamenten zeichnet sich durch eine hohe Bauschigkeit aus, die dem textilen Flächengebilde ein erhöhtes Feuchtigkeitsaufnahmevermögen, Wasseraufnahmevermögen und dergleichen verleiht. Die Feuchtigkeitsaufnahme der kunststoffartigen Filamente, die beispielsweise mit Polyamid-Verbindungen hergestellt sind, ist beispielsweise bei deren Einsatz in textilen Flächengebilden mit verwirbelten Filamenten sehr hoch, kann aber erfahrungsgemäß nur den halben Wert der textilen Flächengebilde mit Baumwollfasern erreichen.

Um das Feuchtigkeitsaufnahmevermögen oder Wasseraufnahmevermögen der textilen Flächengebilde mit kunststoffartigen Filamenten zu erhöhen, werden folglich die Filamente herkömmlichen Texturierungsverfahren unterworfen. Die aus infolge der Texturierungsverfahren texturierten, wie verwirbelten, Filamenten herstellbaren Bauschgarne zeichnen sich nicht nur durch ein hohes Volumen, Bauschigkeit, aus, sondern auch durch eine deutliche Vergrößerung der Oberfläche, die zumindest das Anbinden der Exsudate oder Bestandteile derselben, wie von Wassermolekülen aus Feuchtigkeit, wie Schweiß, Urinfeuchtigkeit, und/oder aus Flüssigkeit, wie Urin, Schweißtropfen, deutlich erhöht.

Unter Bekleidungsstück wird im Sinne der Erfindung auch verstanden jedwedes Kleidungsstück, das unmittelbaren Kontakt, wie mittels Unterwäsche, Schlüpfer, Slips, Unterhemden, usw., oder mittelbaren Kontakt, wie mittels Jacken, Mäntel, usw., mit der Haut des Benutzers beim Tragen desselben aufweist.

Unter textilem Flächengebilde wird im Sinne der Erfindung auch verstanden, ein Gewebe, dass durch das rechtwinkelige Verkreuzen von mindestens zwei Fadensystemen gebildet wird, von denen das eine parallel zur Webkante, das andere quer verlaufen können, wobei das Gewebe Filamente mit kunststoffartigen Polymer-Verbindungen enthalten kann.

Unter textilem Flächengebilde kann im Sinne der Erfindung auch verstanden werden ein Maschenstoff, der im Gegensatz zu dem Gewebe kein Erzeugnis einer Weberei ist, sondern bei welchem eine Fadenschlinge in eine andere Fadenschleife hineingeschlungen ist. Die Masche als mit anderen Schleifen verbundene Schlinge ist die kleinste Einheit, aus der ein Wirk- oder Strickstoff als Maschenstoff hergestellt ist.

Unter textilem Flächengebilde kann auch ein Vliesstoff verstanden werden, der ein textiles Flächengebilde ist, das durch Verkleben und/oder Verschweißen miteinander verbunden ist.

Unter Filamente werden auch im Sinne der Erfindung Fasern oder Garne, wie Filamentgarne, und dergleichen verstanden, die Filamente enthalten. Im Sinne der Erfindung werden unter kunststoffartigen Filamenten werden auch Filamente mit kunststoffartigen Polymer-Verbindungen verstanden.

Im Sinne der Erfindung können unter kunststoffartigen Polymer-Verbindungen auch Polyacrylamid-Verbindungen, Polyprophylen-Verbindungen, Polyester-Verbindungen, Polyacrylnitril-Verbindungen, Polyamid-Verbindungen, Polyacrylimid-Verbindungen und dergleichen sowie Mischungen derselben verstanden werden. Im Sinne der Erfindung können unter kunststoffartigen Polymer-Verbindungen der Einfachheit halber ebenso Naturfasern, Baumwollfasern, Wollfasern, Fasern mit Viskose- Verbindungen, Cellulose-Verbindungen und dergleichen sowie Mischungen derselben verstanden werden.

Die Besonderheit der erfindungsgemäßen Vorrichtung ist auch darin zu sehen, dass an das Bekleidungsstück die erfindungsgemäße Vorrichtung gekoppelt ist, die die Nutzungsdauer der Einlage der erfindungsgemäßen Vorrichtung und damit des Bekleidungsstücks über mindestens eine für die Nutzungsdauer charakteristische Messgröße zur Bestimmung der Nutzungsdauer des Bekleidungsstücks erfasst. Die Messgrößen können sein die Ausmaße von flächenmäßiger Ausbreitung des Farbumschlags auf der Oberseite der Deckschicht, Stärke des Farbumschlags auf der Oberseite der Deckschicht, Tiefe des Farbumschlags in der Deckschicht in Richtung zu deren Unterseite hin, Stärke des Farbumschlags in der Tiefer der Deckschicht, usw. Was die Erfassung der Messgrößen betrifft, so kann die Erfassung in qualitativer und / oder quantitativer Weise stattfinden.

Im Rahmen der Erfindung der erfindungsgemäßen Vorrichtung sind die Messgröße oder Messgrößen infolge der Einwirkung des Exsudates auf die Einlage mit Indikatormittel qualitativ erfassbar, so dass der Umfang der Einwirkungsdauer unerheblich wäre; ebenso sind im Rahmen der Erfindung der erfindungsgemäßen Vorrichtung die Messgröße oder Messgrößen infolge der zeitlichen Länge, also Zeitdauer, der Einwirkung des Exsudates auf die Einlage mit Indikatormittel quantitativ erfassbar; die Zeitdauer kann die einmalige oder mehrmalige Einwirkung umfassen.

Die vorgenannten wie auch nachfolgenden Ausgestaltungen und Ausführungsbeispiele sind in keiner Weise beschränkend und der Fachmann ist jederzeit in der Lage, die entsprechenden textilen und papierenen Flächengebilde und deren Zusammensetzung für die Einlage, z.B. Vliesstoffe mit verwirbelten Filamenten, mit verwirbelten und glatten Filamenten oder mit glatten Filamenten auszuwählen, welche zur Erfassung einer die Nutzungsdauer der Einlage und damit des Bekleidungsstücks charakteristischen Messgröße geeignet sind.

Die durch die Texturierung erzeugten verwirbelten Filamente oder Filamentgarne führen von den ursprünglich glatten Filamenten vor der Texturierung zu verwirbelten, spiralähnlichen Wellen oder zickzackförmigen Strukturen nach Texturierung, sodass ein solcher Faserverband entsteht, dessen Einzelkapillaren sich nur punktförmig mit ihren Strukturbögen berühren. Daraus folgt das große Volumen als das hervorstechende Merkmal solcher texturierten Filamente und Filamentgarne. Das große Volumen ist auch als weitere Folge einer Änderung des Bauschcharakters, welcher sich durch die Feuchtigkeitsdurchlässigkeit oder Feuchtigkeitsaufnahme auszeichnet. Die Feuchtigkeitsaufnahme beispielsweise in der vorliegenden erfindungsgemäßen Einlage ermöglicht die Aufnahme von Exsudaten, wie Urin, des Benutzers, auch wenn das Filament kunststoffartige Polymer-Verbindungen enthält, die an sich hydrophob wären. Die Feuchtigkeitsaufnahme kommt durch die Kapillarkräfte der Schlingen und Kräuselbögen des texturierten Filamente bzw. des textilen Flächengebildes mit texturierten Filamenten zustande.

Da die textilen Flächengebilde mit verwirbelten Filamenten von Flächengebilden mit glatten Filamenten sich in ihrem Feuchtigkeitsaufnahmevermögen als ihre Eigenschaft, Feuchtigkeit oder Flüssigkeit aufzunehmen und zu binden, unterscheiden, zeigt es sich der Vorteil, dass die textilen Flächengebilde, beispielsweise mit textilen glatten Filamenten, einen langen Zeitraum benötigen, um Feuchtigkeit oder Flüssigkeit aufzunehmen, hingegen textile Flächengebilde mit verwirbelten Filamenten einen kurzen Zeitraum benötigen, um Feuchtigkeit oder Flüssigkeit zu binden.

Unter Aufnahme von Flüssigkeit wird im Sinne der Erfindung auch das Binden von Wassermolekülen aus Feuchtigkeit, wie Schweißfeuchtigkeit, und Flüssigkeit, wie Urin, Schweißtropfen, an Oberflächen verstanden.

Auch zeigt sich, dass textile Flächengebilde, die lediglich Gewebe enthalten, ebenso eine bestimmte Zeitdauer benötigen, wenn auch eine sehr lange Zeitdauer, benötigen, um Wassermoleküle aus der Feuchtigkeit und/oder Flüssigkeit aufzunehmen.

Die unterschiedlichen Eigenschaften der textilen Flächengebilde bezüglich der Aufnahme und Bindung von Wassermolekülen aus der Feuchtigkeit oder aus einer Flüssigkeit werden in vorteilhafter geschickter Weise von der Einlage und der erfindungsgemäßen Verwendung der Vorrichtung mit der Einlage genutzt.

Während des Tragens eines Bekleidungsstücks, beispielsweise eines Slips, sondert der Benutzer Exsudate ab. Unter Exsudaten, wie menschlichen Ausscheidungen, werden im Sinne der Erfindung auch Urin, Schweiß, Blut, Stuhl, verstanden, so Urin als Flüssigkeit, Schweiß oder Schweißfeuchtigkeit als dampfförmige Feuchtigkeit, Schweißtropfen als Flüssigkeit, dampfförmige Urinfeuchtigkeit, Kot und dergleichen.

Die Exsudate in Form von Feuchtigkeit gelangen infolge Nutzung des mit der erfindungsgemäßen Vorrichtung gekoppelten Bekleidungsstücks in Berührung mit der Oberseite der Einlage, diffundieren bzw. dringen in die Einlage, wie in deren Inneren, ein und/oder können sich als Niederschlag an der Oberseite der Einlage niederschlagen, können diffundieren bzw. als Flüssigkeit in die Einlage auch eindringen, so dass diese mit der Indikator-Verbindung unter Bildung des Farbumschlags reagieren. Die Reaktion mit der Indikator-Verbindung erfolgt auf der Oberseite der Einlage und in der Einlage, wie in deren Inneren.

Auch bei dem Auftreten von Urin als Exsudat in flüssiger Form infolge Nutzung des mit der erfindungsgemäßen Vorrichtung gekoppelten Bekleidungsstücks gelangt der Urin in Berührung mit der Oberseite der Einlage, diffundiert bzw. dringt in die Einlage, wie in deren Inneren, so dass dieser mit der Indikator-Verbindung unter Bildung des Farbumschlags reagiert. Die Reaktion mit der Indikator-Verbindung erfolgt auf der Oberseite der Einlage und in der Einlage, wie in deren Inneren.

Das Ausmaß des Eindringens des Exsudats -unabhängig von seiner Konsistenz, in die Einlage oder in die Deckschicht als oberer Bereich der Einlage ist auch abhängig von der Zeitdauer, die dem Eindringen des Exsudates gegeben wird. Da das Exsudat mit der Indikator-Verbindung zu einem Farbumschlag reagiert, ist das Auftreten des Farbumschlags ebenso abhängig von der Zeitdauer, die dem Eindringen des Exsudates gegeben wird.

Daher ist auch das Ausmaß des Farbumschlags auf der Oberseite der Einlage, z.B. als flächiger Farbumschlag, und der Farbumschlag in dem Inneren der Einlage, z.B. Tiefenfarbumschlag, ebenso abhängig von der Zeitdauer der Aussetzung oder der Exposition des Exsudates gegenüber der Einlage.

Ebenso zeigt sich, dass das Ausmaß des Eindringens des Exsudats -unabhängig von seiner Konsistenz, in die Einlage oder in die Deckschicht als oberer Bereich der Einlage auch abhängig ist von der Zusammensetzung der oberen Bereich oder der Deckschicht, denn das Exsudat bindet sich verstärkt unter Bildung eines verstärkten Farbumschlags an ein textiles Trägergebildes, welches eine große Oberfläche dem Exsudat anbietet, als ein textiles Trägergebildes, welches eine kleine Oberfläche dem Exsudat anbietet.

Da die Zeitdauer der Exposition des Exsudates gegenüber der Einlage auch das Ausmaß des Farbumschlags und die Zusammensetzung der mit dem Exsudat zu reagierenden Abschnitte der Einlage ebenso das Ausmaß des Farbumschlags bestimmen kann, zeigt sich, dass es vorteilhaft ist, dass die Einlage mindestens zwei Abschnitte des oberen Bereichs oder der Deckschicht aufweist, die sich in der Zusammensetzung voneinander unterscheiden, um infolge Auftretens der unterschiedlichen Farbumschläge auf und /oder in diesen Abschnitten die Dauer der Exposition der Einlage gegenüber dem Exsudat und einhergehend die Dauer der Nutzung des mit der Einlage versehenen Bekleidungsstücks bestimmen zu können.

So kann der erste Abschnitt der Deckschicht ein solches textiles Flächengebilde aufweisen, welches infolge Nutzung des mit der erfindungsgemäßen Vorrichtung gekoppelten Bekleidungsstücks innerhalb eines kurzen Zeitraumes Exsudat zu binden und einen Farbumschlag zu erzeugen vermag. Der erste Abschnitt weißt ein textiles Flächengebilde mit einem Vliesstoff auf, aufweisen, welcher verwirbelte Filamente umfasst. Infolge der hohen Oberflächenvergrößerung des Vliesstoffes wird innerhalb kurzer Zeit Exsudat aufgenommen von dem ersten Abschnitt gebunden, sodass die Zeitdauer vom Beginn des Tragens des Bekleidungsstücks bis zu der Bindung des Exsudates mit Farbschlagerzeugung auf und/oder in dem ersten Abschnitt der erfindungsgemäßen Einlage für eine kurze Zeitdauer der Nutzung spricht.

Der zweite Abschnitt der Deckschicht, der ein textiles Flächengebilde mit einem Vliesstoff aufweist, welcher verwirbelte und glatte Filamente umfasst, kann infolge seiner im Vergleich zu dem ersten Abschnitt geringen oder geringeren Oberflächenvergrößerung des Vliesstoffes innerhalb der kurzen Zeit kein Exsudat oder wenig aufnehmen und binden mit Farbumschlagserzeugung, so dass der geringe Farbumschlag des zweiten Abschnitts für eine kurze Exposition des Exsudates gegenüber der Einlage und damit für eine kurze Dauer der Nutzung des Bekleidungsstücks spricht. So ist es ein Leichtes, aufgrund des Farbumschlags an und/oder in dem ersten Abschnitt und des fehlenden oder nur geringfügig auftretenden Farbumschlags an und/oder in dem zweiten Abschnitt auf die Zeitdauer der Nutzung des mit der Einlage versehenen Bekleidungsstücks zu schließen und quantitativ bestimmen. So kann der Vliesstoff des zweiten Abschnitts 80 Vol.-% oder Gew.-% verwirbelte Filamente und 20 Vol.-% oder Gew.-% glatte Filamente umfassen.

Es ist ohne weiteres möglich, einen Vliesstoff mit texturierten, wie verwirbelten, Filamenten für den ersten Abschnitt bereitzuhalten, der innerhalb einer bestimmten Zeitdauer Exsudat binden kann beispielsweise innerhalb von 5 bis 15 Minuten, vorzugsweise 5 oder 10 Minuten, wie es ebenso ohne weiteres möglich ist, einen Vliesstoff mit einer Mischung mit glatten und texturierten, wie verwirbelten, Filamenten für den zweiten Abschnitt bereitzuhalten, der innerhalb dieser vorbestimmten Zeitdauer kein oder nur wenig Exsudat binden kann, beispielsweise innerhalb von 5 bis 15 Minuten, vorzugsweise 5 oder 10 Minuten. So könnte der zweite Abschnitt erst innerhalb von 15 bis 30 Minuten, vorzugsweise 20 oder 30 Minuten, Farbumschlag zeigen.

Der dritte Abschnitt der Deckschicht, der ein textiles Flächengebilde mit einem Vliesstoff aufweist, welcher verglichen mit dem zweiten Abschnitt weniger verwirbelte Filamente und mehr glatte Filamente gewichts- und/oder volumenmäßig umfasst, kann 20 Vol.-% oder Gew.-% verwirbelte Filamente und 80 Vol.-% oder Gew.-% glatte Filamente umfassen. Infolge des hohen Anteils an glatten Filamenten und des geringen Anteils an verwirbelten Filamenten in dem Vliesstoff zeichnet sich der dritte Abschnitt durch eine -verglichen mit der Oberflächenvergrößerung des zweiten Abschnitts deutlich geringe oder geringere Oberflächenvergrößerung aus. Falls infolge Nutzung des mit der erfindungsgemäßen Vorrichtung gekoppelten Bekleidungsstücks die Einlage dem Exsudat eine Zeitdauer lang ausgesetzt wird, innerhalb welcher -abgesehen von dem Farbumschlag im ersten Abschnitt- an und /oder in dem zweiten Abschnitt der Farbumschlag erzeugt wird, tritt der Farbumschlags an und/oder in dem dritten Abschnitt nicht oder nur geringfügig auf, so dass es ein Leichtes ist, aufgrund des Farbumschlags an und/oder in dem zweiten Abschnitt und des fehlenden oder nur geringfügig auftretenden Farbumschlags an und/oder in dem dritten Abschnitt auf die Zeitdauer der Nutzung des mit der Einlage versehenen Bekleidungsstücks zu schließen und quantitativ bestimmen.

Es ist ohne weiteres möglich, einen Vliesstoff mit texturierten, wie verwirbelten, und glatten Filamenten eines vorbestimmten volumen- oder gewichtsmäßigen Mischungsverhältnisses für den zweiten Abschnitt bereitzuhalten, der innerhalb einer bestimmten Zeitdauer Exsudat binden kann beispielsweise innerhalb von 15 bis 30 Minuten, vorzugsweise 20 oder 30 Minuten, wie es ebenso ohne weiteres möglich ist, einen Vliesstoff mit einer Mischung mit glatten und texturierten, wie verwirbelten, Filamenten für den dritten Abschnitt bereitzuhalten, der innerhalb dieser vorbestimmten Zeitdauer kein oder nur wenig Exsudat binden kann, beispielsweise innerhalb von 15 bis 30 Minuten, vorzugsweise 20 oder 30 Minuten.

Das volumen- oder gewichtsmäßige Mischungsverhältnis des dritten Abschnitts mit texturierten, wie verwirbelten, und glatten Filamenten kann dergestalt ausgebildet sein, dass innerhalb einer bestimmten Zeitdauer z.B. erst mit Ablauf 2 bis 12 Stunden, vorzugsweise 2, 4 oder 6 oder 24 Stunden, der Farbumschlag eintritt.

In einer besonders vorteilhaften Ausgestaltung erstrecken sich die Abschnitte der Deckschicht oder des oberen Bereichs der Einlage von der Oberseite der Deckschicht bzw. oberen Bereichs bis zu der Unterseite von Deckschicht oder von oberen Bereich; die Deckschicht kann des Weiteren dergestalt ausgebildet sein, dass der in der Tiefe der Abschnitte erzeugte Farbumschlag ohne weiteres seitlich, z.B. von zumindest einer Seite oder von beiden, wie gegenüberliegenden, Seiten, erkennbar ist. Gegen die Unterseite der Deckschicht kann die Trägerschicht angesetzt sein, wie an dieser gekoppelt, sein. Die Trägerschicht kann vollflächig auf der Unterseite angeordnet sein. Die Trägerschicht kann gleichfalls mit einem textilen oder papierenen Flächengebilde oder als Folie mit einer kunststoffartigen Polymer-Verbindung hergestellt sein.

Hinzutretend ist es ein Leichtes, die die Oberflächenvergrößerung durch Änderung der Mischungsverhältnisse von mindestens einem Vertreter der glatte Filamente, der stark ondulierten Filamente, der schwach ondulierten Filamente, der verwirbelten Filamente, der gestauchten Filamente, der maschenförmigen Filamente, der spiralförmigen Filamente umfassenden Gruppe an das Erfordernis der Überwachung der Nutzungsdauer anzupassen.

Durch das geschickte Zusammenspiel des ersten Abschnittes mit dem zweiten Abschnitt, vorzugsweise des ersten Abschnittes mit dem zweiten Abschnitts und dem dritten Abschnitt, in dem oberen Bereich oder der Deckschicht wird die zuverlässige Überwachung der Nutzung und der Nutzungsdauer des mit der erfindungsgemäßen Vorrichtung mit der Einlage versehenen Bekleidungsstücks und Schuhwerks bereitgestellt.

Mit der erfindungsgemäßen Vorrichtung mit der Einlage wird eine solche bereitgehalten, die sehr preisgünstig herstellbar ist und die infolge der preisgünstigen Herstellung der erfindungsgemäßen Vorrichtung mit der Einlage sich als Massenware ganz besonders für den Versand an Bekleidungsstücken online anbietet.

Die Zuverlässigkeit und die zuverlässig wiederholbaren Prüfergebnisse der Bestimmung der Nutzungsdauer mittels der erfindungsgemäßen Vorrichtung mit der Einlagen und die geringen Herstellungskosten der erfindungsgemäßen Vorrichtung mit der Einlage drängen geradewegs zu dem Einsatz der erfindungsgemäßen Vorrichtung mit der Einlage in dem massenweisen Versand an Bekleidungsstücken online.

Hinzutretend bedarf es keiner hohen Anforderung an die Geschicklichkeit des Online-Versenders, festzustellen, wie lange die Nutzungsdauer durch den Benutzer betrug; ein Blick auf die Einlage genügt bereits.

Des Weiteren zeigt sich, dass die einfach darstellbare Erhöhung der Anzahl auf mehr als drei Abschnitten zur Absicherung der Ergebnisse der Bestimmung der längeren Nutzungsdauer und/oder der genaueren Bestimmung der Nutzungsdauer beitragen kann.

Überdies zeigt sich, dass der Einsatz von zusätzlichen Abschnitten in einer mittleren Schicht unter der Deckschicht, die an die Deckschicht angrenzt, die genaue Bestimmung der längeren Nutzungsdauer auf simple Weise unterstützen kann.

Das geschickte Zusammenwirken der unterschiedlichen Abschnitte der Deckschicht der erfindungsgemäßen Einlage verhindert hinzukommend wirkungsvoll einen unerlaubten Eingriff des Benutzers oder Trägers in die Deckschicht, denn ein Entfernen eines oder mehrerer Abschnitte dieser Deckschicht ist von außen zweifelsfrei erkennbar.

Daher wird durch die erfindungsgemäße Vorrichtung mit der Einlage eine solche Vorrichtung bereitgehalten, die
zuverlässig
zeitabhängig
die Dauer der Nutzung anzeigt,
einen mechanischen Eingriff des Nutzers, der widerrechtlich oder unerwünscht ist, deutlich anzeigbar macht
unter Beibehaltung der wirtschaftlichen Erfordernisse der kostengünstigen Herstellung der erfindungsgemäßen Vorrichtung mit der Einlage als Massenware.

In weiteren Ausgestaltungen der erfindungsgemäßen Vorrichtung mit der Einlage kann auch der zusätzliche Einsatz von Sensoren vorteilhaft sein, wenngleich bereits die unterschiedliche Ausgestaltung, die Zusammensetzung, der Abschnitte des oberen Bereichs als Deckschicht der erfindungsgemäßen Einlage hinreichend ist, um das Ausmaß der Nutzung von Bekleidungsstücken und Schuhwerken darzutun.

Wegen der technischen Erfolge der erfindungsgemäßen Vorrichtung mit der Einlage und der erfindungsgemäßen Verwendung der Vorrichtung mit derselben könnte unter Umständen den Benutzer des Bekleidungsstücks dazu verführt werden, gleichwohl zu versuchen, die Einlage der erfindungsgemäßen Vorrichtung vor seiner Benutzung des Bekleidungsstücks zu entfernen. Daher ist Aufgabe auch der Erfindung, eine solche erfindungsgemäße Vorrichtung mit der Einlage bereitzustellen, die ebenso auf zuverlässige Weise eine feste Verbindung mit dem Bekleidungsstück bereithält, welche nur dem Befugten die Möglichkeit bietet, die Vorrichtung mit der Einlage zu entfernen .

So können bei Einsatz der erfindungsgemäßen Vorrichtung mit der Einlage als Sicherung in Schlüpfern oder Slips als Kopplungseinrichtung an den Rändern der Seiten der Einlage der erfindungsgemäßen Vorrichtung, die gegenüberliegend sind, vorzugsweise teilkreisförmig ausgebildete, Seitenabschnitte angeformt oder angenäht oder angeklebt sein. Die Seitenabschnitte können mit einem textilen Flächengebilde, wie Gewebe, Vlies, Maschenstoff, und/ oder einem papierenen Flächengebilde hergestellt sein. Die Ränder der Seiten stellen Faltungslinien dar, um die die Seitenabschnitte aus dem Ruhezustand in den Faltzustand in Richtung zu der Außenseite des Schrittbereichs des Schlüpfers hin geschwenkt werden. Die Seitenabschnitte können an der Einlage, wie an deren Trägerschicht oder Deckschicht, angeformt sein. Ebenso können die Seitenabschnitte an der Einlage, wie an deren Trägerschicht oder Deckschicht, gekoppelt, wie mit diesen mittels einfacher leicht entfernbarer Heftstiche oder Wechselstiche vernäht oder verklebt, usw. sein.

Bei Ansetzen der erfindungsgemäßen Vorrichtung mit der Einlage, wie mit der der Deckschicht abgewandten Unterseite derselben oder mit deren an der Deckschicht angeordenten Trägerschicht, gegen die Innenseite des Schrittbereichs eines Schlüpfers als Bekleidungsstück können die beiden Seitenabschnitte um die Faltungslinie in Richtung zu der Unterseite des Schrittbereichs hin geschwenkt werden, sodass die der Einlage oder der Abschnitte abgewandten Spitzenbereiche der Seitenabschnitte unter Bildung der Überlappungszone sich überlappen oder überlagern. Der Bereich der Überlappung oder Überlagerung der Spitzenbereiche der Seitenabschnitte kann miteinander mittels herkömmlicher Klebstoffe verklebt werden.

Ebenso ist es in einer weiteren Ausgestaltung der Einlage und deren Verwendung möglich, dass die Seitenabschnitte aus einem papierenem Flächengebilde gleichfalls im Faltzustand miteinander in der Überlappungszone verklebt werden.

Durch die Verwendung von Seitenabschnitten kann gleichfalls durch den Befugten wirkungsvoll und zuverlässig überprüft werden, ob die erfindungsgemäße Vorrichtung mit der Einlage vor beschädigt oder gar entfernt wurde, um die Nutzungsdauer unerwünschterweise zu erhöhen.

Da es sich zeigt, dass häufig Unterwäsche von Markenherstellern mit deren Marken versehen werden, können gleichfalls die Überlappungszonen der erfindungsgemäße Vorrichtung mit der Einlage mit einer Papierlage überklebt sind; die Papierlage kann als Aufdruck die Marke des Herstellers des Bekleidungsstücks als Abbildung darstellen, um dem Benutzer sowohl visuell infolge des Zerstören des Siegels deutlich vor Augen zu führen, dass das Nutzen des Bekleidungsstücks über den kurzen Zeitraum der Nutzung hinaus unerwünscht ist als auch die Schwelle der Entfernung der Papierlage zwecks unerlaubter Nutzung des Bekleidungsstücks hoch anzusetzen; die Entfernung der erfindungsgemäßen Vorrichtung wird hierdurch nicht nur erschwert, sondern offensichtlich beweisbar gemacht.

Darüber hinaus eignen sich die Verwendung der Papierlage zwecks Überklebung der Überlappungszone und die Verklebung der Überlappungszone besonders, um auf einfache, kostengünstige und zweifelfreie Weise die unerlaubte Entfernung der Papierlage für den Online -Versender nachzuweisen und überprüfbar zu gestalten.

Eine Ausgestaltung der erfindungsgemäße Vorrichtung mit der Einlage und deren erfindungsgemäßen Verwendung ist besonders vorteilhaft, wenn die erfindungsgemäße Vorrichtung mit der Einlage sowohl Seitenabschnitte aufweist als auch mittels einfacher Heftstiche oder Wechselstiche die umlaufenden Ränder der Einlage mit dem Bekleidungsstück mit einem Faden in Kopplungsstellung vernäht sind, so dass beide freien Enden des Fadens unverknotet sind und der Faden über eines seiner beiden Enden herausgezogen werden kann zwecks Entfernung der Einlage im Fall der regulären Nutzung.

Die Trägerschicht kann gleichfalls mit einer kunststoffartigen Polymer-Verbindung hergestellt sein.

Es zeigt sich, dass die erfindungsgemäße Einlage mit
Abschnitten mit unterschiedlicher Zusammensetzung der textilen Flächengebilde, deren Filamente durch Texturierung erwünschte Volumenzunahme, Feuchtigkeitseigenschaften, wie erhöhte Feuchtigkeitsaufnahme, Vergrößerung der Oberfläche besonders ausgebildet sind, und
der Bereitstellung der zuverlässig überprüfbaren Verbindung der Einlage mit dem Bekleidungsstück
dem Befugten, wie Hersteller, Online-Versender und dergleichen
in hinreichender Weise Nachweise verleiht,
ob das Bekleidungsstück lediglich einmalig für eine kurze Zeitdauer oder für eine längere Zeitdauer benutzt wurde.

Auch zeigen Farbumschläge an und/oder in den o.g. Abschnitten die gebührliche oder die ungebührliche Dauer der Nutzung des Bekleidungsstücks an, die Prüfung der Abschnitte gerade im Alltagsgeschäft des Online-Handels überraschend einfach und in hohem Umfang zuverlässig erfolgen kann.

In einer weiteren besonderen Ausgestaltungsform der erfindungsgemäßen Vorrichtung mit der Einlage und deren Verwendung können auch die Abschnitte der Deckschicht zusätzlich hydrophile Filamente aufweisen, die als Kern-Mantel-Filamente ausgebildet sind. Als Kern-Mantel-Filamente finden sich solche, die einen Kern besitzen, der aus vielen tausend Poren und Kanälen besteht, die Wasser in sich speichern können. Beispielsweise ist der den Kern umhüllende Mantel mit wenigen, z.B. zweibillionstel, Millimeter Dicke ein superfeines Häutchen, das von feinsten Haarröhrchen durchzogen ist. Die Filamente sind vorzugsweise porös strukturiert; zudem können sie mit Polyacrylamid-Verbindungen, mit Polyprophylen-Verbindungen, Polyester-Verbindungen, Polyacrylnitril-Verbindungen, Polyamid-Verbindungen und/oder Polyacrylimid-Verbindungen als kunststoffartige Polymer-Verbindungen; auch eignen sich Vliesstoffe mit Viskose - Verbindungen und /oder Cellulose-Verbindungen und/oder mit Baumwollfasern zusätzlich oder alternativ zu den kunststoffartigen -Verbindungen. So können Filamente mit Polyacrylnitril-Verbindungen, Polyamid - Verbindungen und /oder Polyester - Verbindungen wenig Feuchtigkeit bzw. Wasser aus Exsudaten aufnehmen, hingegen Vliesstoffe mit Baumwolle weit mehr Feuchtigkeit aufnehmen.

Die Abschnitte mit hydrophilen Filamenten zeichnen sich zudem aus, dass sie Feuchtigkeit bis zu 30% ihres Eigengewichtes aufnehmen können und die Feuchtigkeit infolge einer vorbestimmten Nutzungsdauer des Bekleidungsstücks für den Benutzer fühlbar machen, so dass der Benutzer auch berührungsgemäß die eine vorbestimmtes Maß hinausreichende Dauer der Nutzung als unangenehm erfahren kann.

Die Kapillarkräfte dieser Röhrchen ziehen die Feuchtigkeit in das Innere oder zumindest auf die Oberfläche an. Durch den Anteil der hydrophilen Filamente in den Abschnitten kann gleichfalls das Ausmaß der Zeitdauer, innerhalb welcher die Bindung von Exsudaten an und/oder in den Abschnitten eintritt, unterstützt werden.

Die Verwendung der hydrophilen Filamente in den Abschnitten ist besonders vorteilhaft, um Flüssigkeit aus Exsudaten mit höherer Konsistenz, wie Kot, an und/oder in die Abschnitte aufzunehmen ist.

Daher eignen sich die erfindungsgemäße Vorrichtung mit der Einlage und die erfindungsgemäße Verwendung derselben
nicht nur zur Bestimmung der Nutzungsdauer mittels Farbumschlag durch Reaktion von z.B. Flüssigkeit aus Urin und /oder Feuchtigkeit aus Schweiß mit der Indikator-Verbindung,
sondern auch zur Bestimmung der Nutzungsdauer mittels Farbumschlag durch Reaktion von z.B. Flüssigkeit aus Kot.

Hierdurch sind der Einsatz der erfindungsgemäßen Vorrichtung mit der Einlage und deren Verwendung völlig unabhängig von der während des Tragens des Bekleidungsstücks auftretenden Art des Exsudates und fördern die ubiquitären Anwendungsmöglichkeiten der erfindungsgemäßen Vorrichtung mit der Einlage und deren Verwendung.

In einer weiteren Ausgestaltung sind die Kern-Mantel-Filamente mit der Indikator-Verbindung versehen; unter Versehen wird auch im Sinne der Erfindung verstanden Tränkung, Beschichtung, Imprägnierung, Bedruckung, Aufpinselung oder Besprühung oder dergleichen.

In einer weiteren Ausgestaltung der erfindungsgemäßen Einlage und Verwendung derselben können statt der hydrophilen Filamente oder zusätzlich zu den hydrophilen Filamente die Abschnitte mit einer Wasser anziehenden oder Flüssigkeit anziehenden Verbindung, wie Polyethylenglykol, versehen werden. Polyethylenglykol wird als toxikologisch unbedenklich eingestuft und ist sogar biologisch abbaubar; die Fähigkeit, Flüssigkeit zu binden oder aufzunehmen, nimmt mit steigender Molekülmasse des Polyethylenglykols ab, so dass mittels der Molekülmasse die Aufnahme von Exsudaten oder deren Bestandteile unterstützt, wie vergrößert oder verkleinert, oder gesteuert werden. So können die Abschnitte mit einem bestimmten Gehalt an Polyethylenglykol versehen sein.

In einer besonders vorteilhaften Ausgestaltung der erfindungsgemäßen Verwendung kann die Schicht ebenso mit einer Polyvinylalkohol-Polymerverbindung beschichtet sein, die in Gegenwart von Flüssigkeit wie Urin, Schweiß sich auflöst und gezielt das Binden von Flüssigkeit aus dem Exsudat an und/oder in den Abschnitten gezielt möglich macht. Durch Versehen von einem oder mehreren Abschnitten mit Polyvinylalkohol-Polymerverbindungen, durch die vorbestimmte Dicke der Schicht, kann das Auflösen der Schicht mit Polyvinylalkohol-Polymerverbindungen ein Maß ist für die Dauer des Kontaktes der Schicht mit Exsudat und daher auch ein Maß für die Dauer der Nutzung des Bekleidungsstücks infolge Exposition gegenüber Exsudat sein.

Im Sinne der Erfindung wird Nutzung des Bekleidungsstücks auch gleichgesetzt die Zeitdauer, z.B. des Berührens der Oberfläche der Einlage oder deren Abschnitte oder dieser Schicht mit Exsudat in flüssiger und/oder dampf - oder feuchtigkeitsförmiger Phase des Exsudats, beispielsweise beim Tragen des Bekleidungsstück durch den Nutzer.

Durch die Verwendung von Vliesstoff, welcher sich durch eine Vergrößerung der Oberfläche auszeichnet, wird vorteilhafterweise die Grenzschicht vergrößert zwischen dem Vliesstoff bzw. zwischen den den Vliesstoff bereitzustellenden Filamenten und Luft. Unter Vliesstoff wird auch ein Textilverbundstoff verstanden, der nicht infolge Verwendung klassischer Methode der Gewebebindung von Kette und Schuss oder durch Maschenbildung aufweist, sondern durch Verschlingung und/oder kursive und/oder adhäsive Verbindungen von Filamenten als Textilfasern hergestellt ist. Vliesstoffe sind z.B. lockere Materialien aus Filamenten, z.B. mit Polyprophylen-Verbindungen, Polyester-Verbindungen, Polyacrylnitril-Verbindungen, Polyamid-Verbindungen und/oder Polyacrylimid-Verbindungen als kunststoffartige Polymer-Verbindungen; auch eignen sich Vliesstoffe mit Viskose - Verbindungen und /oder Cellulose-Verbindungen und/oder mit Baumwollfasern zusätzlich oder alternativ zu den kunststoffartigen -Verbindungen. So können Filamente mit Polyacrylnitril-Verbindungen, Polyamid - Verbindungen und /oder Polyester - Verbindungen wenig Feuchtigkeit bzw. Wasser aus Exsudaten aufnehmen, hingegen Vliesstoffe mit Baumwolle weit mehr Feuchtigkeit aufnehmen.

Das Ausmaß des Farbumschlags in einen Abschnitt der Deckschicht der erfindungsgemäßen Einlage ist auch abhängig von der Diffusion des Exsudates in diesen. Da die Diffusion des Exsudates eine gewisse Zeit benötigt, um in den Abschnitt einzudringen bzw. in dem Abschnitt gebunden zu werden, spricht das Ausmaß des Farbumschlags in dem Abschnitt, dessen Tiefe in den Abschnitt, die Intensität des Farbumschlags, ebenso für die Dauer der Nutzung des Bekleidungsstücks. Ein Abschnitt, der einen Vliesstoff aufweist mit verwirbelten Filamenten, kann innerhalb eines kurzen Zeitraums einen Farbumschlag aufweisen. Ab Beginn des Tragens des Bekleidungsstücks und damit Nutzung desselben durch einen Benutzer wird der Abschnitt mit Exsudat beaufschlagt. Bei kurzer Dauer der Nutzung wird das Exsudat nur oberflächlich, wenn überhaupt, an dem Abschnitt, also an dessen dem Exsudat zu gewandten Oberseite, gebunden und führt zu einem geringen Farbumschlag und wandert nicht oder kaum merklich in den Vliesstoff des Abschnitts. Je länger der Abschnitt dem Exsudat ausgesetzt wird, umso stärker kann der Farbumschlag auf dem Vliesstoff des Abschnitts sich entwickeln, ggf. wird die Fläche des Farbumschlags auf dem Vliesstoff des Abschnitts flächenmäßig größer, und umso tiefer kann auch das Exsudat in den Vliesstoff des Abschnitts einwandem oder diffundieren, was sich durch die Tiefe des diffundierten Farbumschlags in dem Vliesstoff des Abschnitts, die Intensität des Farbumschlags, usw. zeigen kann. Da die Diffusion ein zeitabhängiger Vorgang ist, benötigt das Exsudat mehr Zeit, um in den Abschnitt eindringen und den Farbumschlag zu erzeugen.

Da die Diffusion des Exsudates oder Bestandteile desselben durch die Verwendung von Filamenten mit Polyamid-Verbindungen erhöht werden kann, die Anwesenheit von hydrophilen Kern-Mantel-Filamente zusätzlich zu den Filamenten mit Polyamid-Verbindungen noch beträchtlich gesteigert werden kann, eignet sich besonders ein Abschnitt der Deckschicht zum Nachweis von Exsudat fester Konsistenz, wie Kot, der Vliesstoff mit Filamenten mit Polyamid-Verbindungen und mit hydrophilen Kern-Mantel-Filamente zusätzlich zu den Abschnitten, die zum Nachweis von Urin und Schweiß vorgesehen sind.

Durch die Verwendung der hydrophilen Filamenten und der Filamente mit Polyamid-Verbindungen wird auch der Nachweis der Nutzung des Bekleidungsstücks auch in Abwesenheit von Schweiß und Urin zuverlässig dem Online-Versender bereitgestellt.

Die Indikator-Verbindung kann in einer erfindungsgemäßen Ausgestaltung zudem in Mikrokapseln eingeschlossen sind, welche infolge Kraftbeaufschlagung auf die Einlage zum Beispiel durch das Ansetzen des Bekleidungsstücks gegen an den Körper brüchig werden, so das die Indikatior-Verbindung auszufließen und mit dem Exsuadat zu reagieren vermag. So können die Mäntel oder die Hüllen der Mikrokapseln eine Polyvinylalkohol-Polymerverbindung enthalten, die in Gegenwart von Flüssigkeit aus Exsudat sich auflösen könne. Auch können die Mäntel oder Hüllen eine Polypeptid-Verbindung, z.B. mit einem Molekulargewicht vom 15 000 bis 250.0000 g/mol, wie eine Kollagen-Verbindung, enthalten, welche bei Körperwärme erweichen und bereits bei geringster Feuchtigkeit aufzuquellen vermögen, so dass die Indikator-Verbindung ausfließt und mit dem Exsudat reagiert; es zeigt sich, dass bereits die infolge Erwärmung geringste Brüchigkeit oder Verformbarkeit zum Nachweis einer vorbestimmten Zeitdauer der Nutzung des Bekleidungsstück hinreichend ist.

In einer weiteren erfindungsgemäßen Ausgestaltung weist die Einlage eine Klebschicht auf ihrer unteren Seite auf zwecks Haftung auf dem Abschnitt im Schrittbereich oder Achselbereich; die Indikator-Verbindung und der Sensor können in voneinander beabstandeten Abschnitten der Einlage sich befinden. Die erfindungsgemäße Verwendung der Vorrichtung dient zur Bestimmung der durch einen Benutzer erfolgten Nutzung eines Ober- und Unterbekleidungsstückes und Schuhwerks mit einer oder mehreren Einlagen zur Anordnung auf einem dem Benutzer zugewandten innenseitigen Abschnitt, wie Innenseite, im Schritt- oder Achselbereich des Bekleidungsstücks oder auf einem dem Benutzer zugewandten innenseitigen Abschnitt, wie Innenseite, des Schuhwerks zum Auffangen von Urin, Schweiß und/oder Blut.

Der obere Bereich der Einlage kann mit einem Vliesstoff mit aneinander haftenden Fasern zur Aufnahme der Ausscheidung ausgebildet sein. In einer weiteren Ausgestaltung der erfindungsgemäßen Vorrichtung weist die Einlage einen Schichtaufbau auf, so dass das Eindringen der Ausscheidung aus dem oberen Bereich in den an den oberen Bereich sich anschließenden mittleren Bereich der Einlage der erfindungsgemäßen Vorrichtung erschwert wird und zeitverzögernd erfolgt. So kann mittels der Anordnung von Schichten unterschiedlichen Aufsaugvermögens aufeinander das Eindringen der Ausscheidung zeitverzögernd stattfinden und dieses kann ein Maß der Nutzungsdauer sein; als Schichten eignen sich textile oder papierenes Flächengebilde unterschiedlicher hydrophiler Eigenschaft, die dem Fachmann bekannt sind. So kann durch ein vorbestimmtes Mischungsverhältnis hydrophiler und hydrophober Fasern, mit hydrophilem und hydrophobem Überzug versehener Fasern und/oder eine Anordnung von vorbestimmten Schichten hydrophiler und hydrophober Eigenschaften das Aufsaugvermögen der Einlage auf die Ausscheidungen, deren Zusammensetzung, Eindringvermögen, die Schnelligkeit des Aufsaugvermögens derselben, -und damit auch das zeitabhängige Eindringen- gesteuert sein.

In einer anderen Ausgestaltung der erfindungsgemäßen Vorrichtung weist der obere Bereich der Einlage Abschnitte auf, wobei das Aufsaugvermögen der Abschnitte so ausgebildet ist, dass die Abschnitte zeitverzögernd flächig die Ausscheidung aufsaugen, so dass das Maß der ganzflächigen Aufsaugung der Oberseite des Abschnitts als Maß der Exposition der Einlage gegenüber Ausscheidung gewertet werden kann. In einer ganz anderen Ausgestaltung der erfindungsgemäßen Vorrichtung weist der obere Bereich der Einlage Abschnitte auf, die voneinander beabstandet sein können und die Indikatormittel aufnehmen.

Als Indikatormittel können solche Indikator-Verbindungen verwendet werden, die der Fachmann kennt und derart herstellt, dass sie gesundheitlich unbedenklich sind. Indikator-Verbindungen erzeugen einen Farbumschlag, der bei der Reaktion mit mindestens einer für z.B. menschlichen Schweiß oder Urin charakteristischen Substanz sich zeigt..

Da der Farbumschlag aufgrund der Reaktion mit mindestens einer für das Exsudat charakteristischen Substanz erfolgt, wird in den vorhergehenden und nachfolgenden Beispielen unter Reaktion oder Umsatz von Flüssigkeit von Exsudat oder Schweiß von demselben mit der Indikator-Verbindung die Reaktion mit den in diesen enthaltenden Substanzen im Sinne der Erfindung auch verstanden.

So können als Indikator-Verbindungen Helianthin- (Methylorange), Bromphenolblau-, Kaliumpermanganat-, Phenolphthalein-, Lackmus- Verbindungen oder Mischungen derselben verwendet werden, die den pH-Wert der in dem oberen Bereich der Einlage aufgenommenen Ausscheidungen anzeigen. Infolge Kontakt mit Flüssigkeit des Exsudates und deren Substanzen reagieren die in dem textilen Flächengebilde befindlichen Indikator-Verbindungen mit den Substanzen des Exsudates zu Reaktionsprodukten unter Farbumschlagsbildung. So können auch die Indikator-Verbindungen sich zuerst teilweise in dem textilen Flächengebilde zu Lösung verflüssigen mit anschließender oder gleichzeitiger Reaktion zu Reaktionsprodukten unter Farbumschlagsbildung. Es zeigt sich auch, das bereits Feuchtigkeit genügt, die in das textile Flächengebilde eindringt bzw. diffundiert, um die in diesem befindlichen Indikator-Verbindungen mit den Substanzen zu Reaktionsprodukten unter Farbumschlagsbildung reagieren zu lassen. Als eine der Messgrößen der Ausscheidungen wird der pH-Wert und dessen Veränderung beigezogen; es zeigt sich, dass die Ausscheidungen durch die Aufnahme in den oberen Bereich der Einlage mit der Indikator-Verbindung in Berührung kommen und infolgedessen der pH-Wert erfasst und ein Farbumschlag oder -wechsel der Indikator-Verbindung angezeigt werden. Das Auftreten des Farbumschlags bzw. der Farbumschläge korreliert mit der Anwesenheit des Exsudates bzw. dem Aussetzen der erfindungsgemäßen Vorrichtung gegenüber demselben durch Tragen des Bekleidungsstücks mit der erfindungsgemäßen Vorrichtung.

Das Ausmaß des flächigen Farbumschlags auf der Oberseite des die Indikator-Verbindung aufgenommenen Abschnitts korreliert mit der Dauer des Kontaktes der Einlage mit den Ausscheidungen, da die Abschnitte lediglich zeitverzögernd flächig die Ausscheidung aufzusaugen vermögen.

Darüber hinaus hilft ein Vergleich einer Farbskala mit dem Farbumschlag, der Intensität desselben sowie mit dessen flächigen Ausmaß, um die Nutzung des Bekleidungsstücks festzustellen; das Ausmaß des flächigen Farbumschlag der Indikator-Verbindungen ist ein Maß für die Nutzung oder Abnutzung des Bekleidungsstücks und deren Zeitdauer. Auch das Überschreiten eines Farbschwellenwerts der Farbskala spricht für die Menge an aufgesaugter Ausscheidung, die dem kurzzeitigen Anprobieren des Bekleidungsstückes widerspricht.

Des Weiteren hilft der Vergleich einer Farbskala mit dem Farbumschlag, der Intensität desselben sowie mit dessen flächigen Ausmaß, um die Dauer der Nutzung des Bekleidungsstücks festzustellen; das Ausmaß des flächigen Farbumschlag der Indikator-Verbindungen kann ein Maß für die Abnutzung des Bekleidungsstücks und deren Zeitdauer sein

Zudem ist das Eintreten des Farbumschlags der Indikator-Verbindung in einem Abschnitt des oberen Bereichs der Einlage der qualitative Nachweis für das zumindest einmalige Anprobieren des Bekleidungsstücks; das Ausmaß des Auftretens des flächigen Farbumschlags der Indikator-Verbindung in seinem Abschnitt des oberen Bereichs der Einlage kann hingegen der quantitative Nachweis für die Zeitdauer sein, innerhalb welcher das Bekleidungsstück benutzt wurde. Die Farbumschlag und sein flächiges Ausmaß sind bleibend, so dass der Nachweis des charakteristischen pH-Wertes als Messgröße dauerhaft ist und ohne Zweifel für die ggf. Abnutzung des Bekleidungsstücks infolge deren Dauer einwandfrei und zuverlässig steht.

Auch können kann die Nutzung und/oder die Dauer der Nutzung des Bekleidungsstücks bei Phenolphthaleinlösung als Indikator-Verbindung eine Rotfärbung infolge des Kontaktes mit Schweiß eintreten lassen. Unter Phenolphthaleinlösung oder Lösung von Phenolphthalein wird im Sinne der Erfindung auch die infolge Kontakt mit Exsudatflüssigkeit zumindest teilweise verflüssigte oder angefeuchtete Indikator-Verbindung verstanden. Die qualitative Erfassung der Nutzung wird durch das Auftreten des Farb-umschlag oder -wechsels der Phenolphthaleinlösung als Indikator-Verbindung bestimmt; die quantitative Erfassung der Dauer der Nutzung kann durch die Farbintensität des Farbumschlags von Phenolphthaleinlösung in dem mittleren Bereich der Einlage, z.B. als kräftige Rotfärbung, festgestellt werden, da das Eindringen des Schweißes oder Urins von dem oberen Bereich der Einlage in deren mittleren Bereich zeitverzögernd erfolgt. Die erfindungsgemäße Verwendung kann auf die qualitative Erfassung der Nutzung und/oder die quantitative Erfassung der Dauer der Nutzung durch das geschickte Zusammenwirken von Einlage, deren Ausgestaltung, Indikatormittel gerichtet sein.

Phenolphthaleinlösung ruft bei pH-Werten von 8,4 bis 10,0 eine karminrote Farbe hervor, die bei Vorhandensein von saurem Schweiß rückgängig gemacht wird, so dass schon geringe Farbänderung oder -wechsel ein guter Anzeiger ist für menschlichen Schweiß oder Urin und ggf. das Ausmaß der Farbänderung, dessen flächige Ausbreitung in die Breite des Abschnitts und die Tiefe der Einlage ein Maß für die Menge des aufgenommen Schweißes in der Einlage gegenüber Schweiß und Urin und Nutzung sein können, da ein Benutzer erfahrungsgemäß eine vorbestimmte Menge innerhalb einer vorbestimmten Zeitdauer absondert. Phenolphthaleinlösung ruft bei pH-Werten von 8,4 bis 10,0 eine karminrote Farbe hervor, die bei Vorhandensein von saurem Schweiß rückgängig gemacht wird, so dass schon geringe Farbänderung oder -wechsel ein guter Anzeiger ist für menschlichen Schweiß oder Urin und ggf. das Ausmaß der Farbänderung, dessen flächige Ausbreitung in die Breite des Abschnitts und die Tiefe der Einlage ein Maß für die Menge des aufgenommen Schweißes und die Dauer der Aussetzung der Einlage gegenüber Schweiß und Urin, da ein Benutzer erfahrungsgemäß eine vorbestimmte Menge innerhalb einer vorbestimmten Zeitdauer absondert.

Auch können polymere Verbindungen von 7-Hydroxy-3-phenoxazinon-Chromophoren zur Erfassung der Messgröße des pH-Wertes als Indikator-Verbindung in einem Abschnitt des oberen Bereichs und mittleren Bereichs der Einlage, die als Lackmus bei pH-Werten von 4,5 rot bei 8,3 eine blaue Farbe anzeigen.

Der Farbumschlag, das flächige Ausmaß des Farbumschlags in der Breite des Abschnitts und das in die Tiefe der Einlage sich erstreckende Ausmaß des Farbumschlags in dem mittleren Bereich der Einlage oder gar in dem in Kopplungsstellung der Einlage dem Bekleidungsstück zugewandten unteren Bereich der Einlage können durch herkömmliche, wie physikalische, Messmethoden, z.B. mittels Photometrie, bestimmt werden.

Auch können dem Fachmann bekannte herkömmliche sonstige Indikator-Verbindung eingesetzt werden, die auf Anwesenheit von Substanzen, wie Harnstoff, Harnsäure, Fettsäuren, Kalium, Bicarbonat, Ameisensäure, Buttersäure, Cholesterin, Immunglobuline, Eiweiß, Aminosäuren, Hämoglobin, Ketonen, die für die menschlichen Ausscheidungen, wie Urin, Blut, Kot, Schweiß, usw. spezifisch bzw. charakteristisch sind und mit Farbumschlag reagieren. Schon der optisch ohne Weiteres ersichtliche einfache Farbvergleich der Farben in deren flächiges oberflächliches Ausbreiten in ihren Abschnitten der oberen Bereiche vor dem Tragen und nach dem Tragen des Bekleidungsstückes lässt bereits -unabhängig von aufwendigen herkömmlichen Messmethoden- im Rahmen einer jedermann zugänglichen Grobsichtung erkennen, ob die Nutzung des Bekleidungsstückes erfolgte oder ob Nutzung des Bekleidungsstückes die Dauer des Anprobierens desselben überschritt.

Um auf die Individualität der Ausscheidungen von Benutzern abzustellen, sind die Verwendung mehrerer Messgrößen und deren Erfassung in Urin, Schweiß, und Kotresten usw. mittels der Einlage der erfindungsgemäßen Vorrichtung vorteilhaft. Daher eignet sich eine Vielzahl an Indikator-Verbindungen, die jede für sich in einem Abschnitt der Einlage durch Aufbringen, wie Tränkung, Auftupfen, Aufpinseln, verbracht ist.

Ebenso eignet sich eine Indikator-Verbindung in Kombination mit einem Enzym, welches eine in Ausscheidungen vorhandene Substanz umwandelt, so dass deren Endprodukt als Messgröße infolge Farbumschlag gefasst wird. So kann mittels Urease aus Harnstoff im Urin CO² und Ammoniak eigesetzt werden, der freigesetzte Ammoniak mittels der Indikator-Verbindung optisch aufgrund dessen Farbumschlag erfasst werden.

Auch können Indikator-Verbindungen verwendet werden, die erst nach dem Rückversand des Bekleidungsstücks den Farbumschlag zeigen infolge des nachträglichen Aufsprühens eines weiteren Stoffes, wobei jedoch zuvor infolge der aufgesaugtem Ausscheidung die Indikator-Verbindung reagierte.

In einer vorteilhaften Ausgestaltung der erfindungsgemäßen Vorrichtung nimmt deren Einlage eine elektronische Sensoreinrichtung auf, die einen die Dauer der Nutzung des Bekleidungsstücks über mindestens eine für die Dauer charakteristische Messgröße erfassenden Sensor zur Bestimmung der Abnutzung des Bekleidungsstücks umfasst und die elektronische Sensoreinrichtung einen Sensor mit einer Empfindlichkeit für für menschliche Ausscheidungen charakteristische Substanzen aufweist, die Sensoreinrichtung von dem Sensor ausgebbaren und/oder erfassten Messgrößen korrespondierende oder korrelierende Sensorsignale prozessierbar, wie detektierbar, speicherbar, anzeigbar und/oder ausgebbar macht. Es zeigt sich, dass zwar das Auftreten von Schweißgeruch in neuen Bekleidungsstücken für die Nutzung, wenn nicht gar für die eine bestimmte Zeitdauer lang erfolgte zur Abnutzung führende Nutzung desselben bekannt ist, aber das Prinzip der Erfindung optische Indikatormittel zusammen mit elektronischen Sensoreinrichtung zur gemeinsamen Bestimmung der Abnutzung des Bekleidungsstücks und dessen Dauer gemäß der erfindungsgemäßen Vorrichtung auszugestalten, ist nicht bekannt. Die von der elektronische Sensoreinrichtung erfassbaren Messgrößen können chemische, biologische, biochemische sein, wie pH-Wert, Kochsalz (NaCL), Harnstoff, Harnsäure, Fettsäuren, Kalium, Bicarbonat, Ameisensäure, Buttersäure, Cholesterin, Immunglobuline, die Substanzen enthalten, die für die menschliche Ausscheidungen spezifisch bzw. charakteristisch sind.

Diese Messgrößen sind diejenigen, die auf die Einlage der erfindungsgemäßen Vorrichtung aufgrund der Nutzung des Bekleidungsstücks durch deren Aufnahme einwirken und die Sensoren veranlassen, aufgrund der Erfassung der Messgrößen Sensorsignale zu erzeugen. Die Messgrößen können z.B. Substanzen im Schweiß, wie Ameisensäure, Buttersäue, die Sensoren zur Induktion von Sensorsignalen veranlassen. Auch die Menge der so erfassten Messgröße veranlassen die Erzeugung von Sensorsignalen durch die Sensoren, die unterschiedliche Empfindlichkeiten aufweisen.

Auch können die Sensoren die Dauer der Nutzung, das Ausmaß des Eindringens der Messgröße, z.B. in den mittleren oder den unteren Bereich, in die Einlage, erfassen, so dass nachweisbar die Sensorsignale für den Ort der Sensoren, die Lage der Sensoren in Breite und Tiefer der Einlage, und überprüfbar stehen. Des Weiteren kann die Menge der zu erfassenden Messgrößen mit der Zeitdauer des Tragens des Bekleidungsstückes in Beziehung gesetzt werden, überdies können die Anzahl der Intervalle zwischen der Nutzung und der Nichtnutzung mit der Häufigkeit des Auftretens der Messgrößen und dessen Einwirken auf den Sensor korreliert sein, so dass die Sensorsignale ein sehr genaues Abbild der Nutzung des Bekleidungsstückes und der Dauer der Nutzung widerspiegeln.

In einer Ausbildung der erfindungsgemäßen Vorrichtung ist die elektronische Sensoreinrichtung als elektronisches Bauelement oder IC Baustein ausgestaltet, welches die chemische oder biochemische oder physikalische Messgrößen in der Einlage erfasst und an einen Empfänger weiterleitet. Die vorgenannten und nachfolgend beschriebenen Ausgestaltungen sind nicht in irgendeiner Weise beschränkend, sondern vielmehr ist der Fachmann in der Lage, solche elektronischen Sensoreinrichtungen mit Sensoren bereitzustellen, die sich durch ihre hinreichende Empfindlichkeit gegenüber den in Ausscheidungen zu findenden Substanzen auszeichnen und diese als Messgrößen erfassen, so dass einwandfrei die die Dauer der Nutzung von dem kurzzeitigen Anprobieren eines Bekleidungsstückes zuverlässig unterschieden werden kann.

Die Messgröße wird von der elektronischen Sensoreinrichtung erfasst, indem ein durch eine Reaktion mit den Substanzen der Ausscheidung hervorgerufenes Sensorsignal erzeugt und das Sensorsignal an den Empfänger der elektronischen Sensoreinrichtung weitergeleitet wird; der Empfänger vergleicht das Sensorsignal als Ist-Wert mit in ihm eingegebenen Sollwerten und erzeugt nach Maßgabe der Sollwerte ein mit dem Sensorsignal korrespondierendes Ausgangssignale und leitet dieses an eine Speichereinrichtung weiter zur Speicherung und Ablesen.

Vorzugsweise ist die elektronische Sensoreinrichtung mit einem herkömmlichen RF-ID-(Radio Frequency Identification-Unit) Transponder zur kontakt- oder drahtlosen Übertragung der in der Speichereinrichtung gespeicherten Ausgangssignale ausgebildet, die einen mit der Speichereinrichtung verbundenen Prozessor in einem Siliziumchip, eine Datenübertragungseinrichtung, insbesondere einen Modulator, eine integrierte Spule umfassen, die mit dem Kondensator und dem Siliziumchip einen Schwingkreis bildet, dessen Resonanzfrequenz auf eine bestimmte Frequenz abgestimmt ist; die Spule, welche eine elektrische Leiterschleife mit einer oder mehreren Windungen ist, wird für den Leseprozess des kontaktlosen Transpondersystems herkömmlicherweise in das magnetische Wechselfeld einer Sendeantenne gebracht, so dass eine induktive Kopplung zwischen dem Sender und der Antennenspule aufgebaut wird. Durch das magnetische Wechselfeld wird in der Spule eine elektrische Spannung induziert, welche gleichgerichtet zur Spannungsversorgung eines integrierten Transponderschaltkreises (Transponder-ICs bzw. RFID-Chips) verwendet wird.

Obwohl der RF-ID Transponder zwar miniaturisiert ist, zeigt sich die Ausgestaltung der elektronischen Sensoreinrichtung mit einem modifizierten der RF-ID Transponder als bevorzugt, da ein flexibler fadenartiger elektrischer Leiter benutzt wird, welcher zumindest eine Anschlussstelle für den Prozessor aufweist und mit diesem elektrisch verbunden ist und als Faden in der Einlage aus Gewebe mit im rechten Winkel sich kreuzenden Fäden in Form von Kette und Schuss angeordnet ist. Da der elastische Leiter wegen seines fadenartigen Aufbaus als Antennenspule des Transponders dient, wird die Verformbarkeit der Einlage nicht beeinflusst. Von Vorteil ist ein Leiter mit elektrisch leitfähigen Schuss- und elektrisch leitfähigen Kettfäden, welche an ihrem Kreuzungspunkt elektrisch leitfähig miteinander verbunden sein könne; da der Leiter zudem als Antennenspule den Transponder herstellbar ist und Teil des Gewebes der Einlage ist, schränkt der Leiter vorteilhafterweise das Tragegefühl des Benutzers nicht ein.

Die Einlage der erfindungsgemäßen Vorrichtung kann mit dem Bekleidungsstück über eine solche Naht verbunden sein, dass das in der Naht geführte Garn auf einfache Weise aus der Naht herausgezogen werden kann und die Einlage gelöst ist, das Wiedereinführen des Garns ist undurchführbar.

In einer Ausbildung der erfindungsgemäßen Vorrichtung ist die elektronische Sensoreinrichtung mit einer akustomagnetischen Artikelsicherung (58 KHz) verbunden,; die AM (Akusto-Magnet) Technologie bietet den Einsatz von relativ kleinen bzw. leichten Hart-, Soft- und Klebeetiketten bei großen Durchgangsbreiten mit den CPID-AM Schleusen- oder Monosystemen. Hohe Detektionsraten und fehlalarmfreie Funktion zeichnen AM-Sicherungssysteme aus. Anders als bei der radiofrequenten Technologie können die Hartetiketten nicht durch Metallabdeckung abgeschirmt werden. Klebeetiketten können deaktiviert und reaktiviert werden. Daher eignet sich die AM-Technologie hervorragend zur Teilnahme am Quellensicherungsverfahren zahlreicher Hersteller und Lieferanten ihrer Artikel.

Klebeetiketten werden am POS kontakt- oder distanzdeaktivert. Diese Technologie ist für alle Sortimentsbereiche geeignet. In einer weiteren Ausbildung der erfindungsgemäßen Vorrichtung ist die elektronische Sensoreinrichtung mit Ortungssystemen verbindbar.

In einer anderen Ausbildung der erfindungsgemäßen Vorrichtung ist die Einlage mit dem dem Benutzer zugewandten innenseitigen Abschnitt des Bekleidungsstücks, wie Schritt- oder Achselbereich, mittels eines einfachen Heftstichs oder Wechselstichs vernäht; zum Beispiel wird der umlaufende Rand der Einlage mit dem Abschnitt mittels eines Faden in Form des einfachen Heftstiches vernäht, so dass beide freien Enden des Fadens unverknotet sind und der Faden über eines seiner beiden Enden herausgezogen werden kann zwecks Entfernung der Einlage im Fall der regulären Nutzung. Im Sinne der Erfindung wird unter dem dem Benutzer zugewandten innenseitigen Abschnitt des Bekleidungsstücks auch verstanden, dass das Bekleidungsstück zum herkömmlichen Tragen auf rechts gedreht ist. Da die Einlage als Befestigungsmittel randständig mit dem Bekleidungsstück mittels einer Naht vernäht wird, welche mit einem herausziehbaren Garn zwecks Auflösung derselben ausgebildet ist, ist das zwischenzeitliche Entfernen der Einlage zwecks Aufrechterhaltung der Dauer der Nutzung unmöglich gemacht.

Auf der Oberseite der Einlage, z.B. auf der Oberseite der Deckschicht, ist vorteilhafterweise eine Beschichtung angeordnet, die als Oberschicht auf der Deckschicht als Ober- oder Außenschicht aufliegt und/oder die Deckschicht oberseitig mit der Beschichtung versehen, wie bedampft, getränkt, sein kann. Die Beschichtung kann als eine wasserdampfdurchlässige flüssigkeitsundurchlässige Schicht ausgestaltet sein, so dass zwar Dämpfe, wie sie infolge der Anwesenheit von Harn, Körpersekret, Schweiß, usw., vorliegen, die Beschichtung bzw. die Schicht durchdringen können und mit den Indikatormitteln der Einlage zu den Farbumschlag erzeugenden Reaktionsprodukten zu reagieren vermögen, aber die Reaktionsprodukte nicht durch die Beschichtung in Richtung zu der mit dem Körper des Benutzers in Kontakt gelangbaren Oberseite dringen, um deren Kontakt mit der Haut des Benutzers zu vermeiden, zumal die Reaktionsprodukte in der Einlage flüssig oder fest verbleiben können. Auch kann die Deckschicht oberseitig mit mehreren Beschichtungen versehen, wie getränkt, sein.

Da die Möglichkeit besteht, dass die Einlage der erfindungsgemäßen Vorrichtung mittels eines als herkömmliche Binde gestalteten Schritteinsatzes während der unerlaubt dauerhaften Nutzung bedeckt wird, um die Aufnahme von Exsudat in diese zu vermeiden, kann die Beschichtung auch ein Polypeptid, z.B. mit einem Molekulargewicht vom 15 000 bis 250.0000 g/mol, wie eine Kollagen-Verbindung, enthalten, welches bei Körperwärme erweicht und bereits bei geringster Feuchtigkeit aufzuquellen vermag; es zeigt sich, dass bereits die infolge Erwärmung geringste Verformbarkeit der Beschichtung genügt zum Nachweis eines Negativabdrucks der Unterseite einer herkömmlichen Binde. Die Beschichtung wird infolge des Tragens des Bekleidungsstücks erwärmt und erweicht, so dass die Oberfläche der Beschichtung durch die Auflage der Binde in seiner Oberflächenstruktur sich verändert.

Die wasserdampfdurchlässige flüssigkeitsundurchlässige Schicht kann als eine Trennschicht ausgebildet sein, die mindestens abschnittsweise mit der Einlage verbunden sein kann. Die Einlage kann mit Polymeren, z.B. auf Cellulosegrundlage, zur Bereitstellung der Trennschicht beschichtet oder getränkt sein, dass mindestens diese ganzflächig von diesen Polymeren bedeckt ist. Ganzflächig bedeutet, dass die gesamte Oberfläche mindestens einer Seite der Einlage diese Polymeren aufweist, so dass für den Durchgang von Reaktionsprodukten geeignete sogenannte Fehlstellen auf der Oberfläche der Einlage nicht vorhanden sind. Die Trennschicht als eine Membran mit Polymeren auf Polyetherestergrundlage, vorzugsweise Copolyetherestergrundlage, Polymeren auf Polypropylengrundlage und / oder Polymeren auf Polyetheramidgrundlage ausgebildet sein. Unter Membran wird auch ein Film ohne Fehlstellen oder Folie verstanden. Zudem kann auch eine, vorzugsweise mikroporöse, Membran verwendet werden, die Polymere wie Polyolefine, Polyethylen-Polypropylen-Copolymere, Polyethylen, Terephthalate, Polycaprolactam, Polyvinylidenfluorid, Polybutylenterephthalat, Polyestercopolymere und Polytetrafluorethylen umfassen kann. Durch Tränkung, Auftragen, Bestreichen oder Beschichtung mit Polymeren auf Polyetherestergrundlage, vorzugsweise Copolyetherestergrundlage, Polymeren auf Polypropylengrundlage und / oder Polymeren auf Polyetheramidgrundlage kann die Trennschicht auf mindestens einer Seite der Einlage angeordnet sein. Die Membran bzw. Trennschicht etc. kann leicht mit üblichen Klebern an die oder auf der Einlage geklebt werden, wobei die mit Kleber benetzte Fläche der Membran in einer bevorzugten Ausführungsform, z.B. eine cellulosische, selektiv permeable Membran, mit Polyurethankleber mindestens einseitig mit einem textilen Gewebe der Einlage zumindest abschnittsweise, bevorzugt in einem regelmäßigen Punktraster, verklebt ist. Vorzugsweise ist die Membran durchsichtig ausgestaltet zwecks Feststellung von Farbumschlägen.

### Ausführungsbeispiele

Die Ausführungsbeispiele offenbaren eine Vereinfachung in schematischer, stark vergrößerter Weise, ohne Anspruch auf eine maßstabsgetreue Wiedergabe, Ausführungsformen und Weiterentwicklung ohne Beschränkung der Erfindung.

Die erfindungsgemäße Vorrichtung hat in einem Ausführungsbeispiel eine Einlage mit Indikatormitteln, die eine Indikator-Verbindung und eine elektronische Sensoreinrichtung mit einem Sensor zur Bestimmung der Abnutzung des Bekleidungsstücks umfasst. Die Indikator-Verbindung zeigt infolge ihrer Reaktion mit für menschliche Ausscheidungen charakteristischen Substanzen einen Farbumschlag zeigt und der Sensor der elektronischen Sensoreinrichtung erzeugt infolge des Kontaktes mit einer für menschliche Ausscheidungen charakteristischen Substanz sin Sensorsignal. Der Sensor erfasst eine die Dauer der Nutzung des Bekleidungsstücks über mindestens eine für die Dauer charakteristische Messgröße. Als Indikatormittel wird Kaliumpermanganat für die Erfassung des pH-Wertes als Messgröße.

In einem Ausführungsbeispiel weist die Einlage ein textiles Flächengebilde mit Helianthin als Indikator-Verbindungen auf, deren oberer Bereich einen Abschnitt einen Vliesstoff mit verwirbelten Filamenten hat.

In einem weiteren Ausführungsbeispiel weist die Einlage eine Deckschicht mit drei Abschnitten auf, die sich von der Oberseite der Deckschicht bis zu deren Unterseite erstrecken. Gegen die Unterseite der Deckschicht ist eine Trägerschicht angesetzt und flächig auf der Deckschicht aufliegend. Der erste Abschnitt umfasst einen Vliesstoff mit verwirbelten Filamenten, der zweite Abschnitt hat einen Vliesstoff mit verwirbelten und glatten Filamenten und der dritte Abschnitt weist einen Vliesstoff mit glatten Filamenten auf. In den Vliesstoffen befindet Helianthin als Indikator-Verbindung, in anderen Ausführungsbeispielen Bromphenolblau, Kaliumpermanganat, Phenolphthalein oder Lackmus. Die Einlage ist mit dem Schrittbereichs des Schlüpfers als Bekleidungsstück mittels eines Heftstichs oder eines Wechselstichs vernäht. Da die Einlage als Befestigungsmittel randständig mit dem Bekleidungsstück mittels einer Naht vernäht wird, welche mit einem herausziehbaren Garn zwecks Auflösung derselben ausgebildet ist, ist das zwischenzeitliche Entfernen der Einlage zwecks Aufrechterhaltung der Dauer der Nutzung unmöglich gemacht.

Aufgrund der unterschiedlichen Oberflächenvergrößerung der Vliesstoffe zeigt es sich, dass in und auf den Abschnitten unterschiedlich schnell Farbumschläge nacheinander infolge der Reaktion der Indikator-Verbindungen mit den in dem Harn als Exsudat befindlichen Substanzen auftreten in Abhängigkeit von der Exposition (Aussetzen) der Einlage gegenüber dem Exsudat. Der Farbumschlag in und auf dem ersten Abschnitt erfolgt bereits rasch nach einer Zeitdauer von ca. 5 bis 15 min, der in und auf dem zweiten Abschnitt tritt nach ca. 20 min und der in und auf dem dritten Abschnitt tritt erst nach 3 Stunden auf.

In einem ganz anderen Ausführungsbeispiel weist die Einlage eine Deckschicht mit drei Abschnitten auf, die sich von der Oberseite der Deckschicht bis zu deren Unterseite erstrecken. Gegen die Unterseite der Deckschicht ist eine Trägerschicht angesetzt und flächig auf der Deckschicht aufliegend. die Einlage mit dem Bekleidungsstück mittels eines Heftstichs oder eines Wechselstichs vernäht wird. Die Trägerschicht ist unter Verwendung von Kautschuk flüssigkeitsdicht ausgestaltet ist. Da die Einlage als Befestigungsmittel randständig mit dem Bekleidungsstück mittels einer Naht vernäht wird, welche mit einem herausziehbaren Garn zwecks Auflösung derselben ausgebildet ist, ist das zwischenzeitliche Entfernen der Einlage zwecks Aufrechterhaltung der Dauer der Nutzung unmöglich gemacht. Der erste Abschnitt umfasst einen Vliesstoff mit verwirbelten Filamenten, der zweite Abschnitt hat einen Vliesstoff mit verwirbelten und glatten Filamenten und der dritte Abschnitt weist einen Vliesstoff mit glatten Filamenten auf. In den Vliesstoffen befindet Helianthin als Indikator-Verbindung, in anderen Ausführungsbeispielen Bromphenolblau, Kaliumpermanganat, Phenolphthalein oder Lackmus. Auf der Oberseite der Deckschicht ist eine die Polyvinylalkohol-Polymerverbindung enthaltende Außenschicht angeordnet. Aufgrund der Exposition der Einlage gegenüber Exsudat wird die Außenschicht infolge Körperwärme des Benutzers und der Flüssigkeit des Exsudates zumindest teilweise aufgelöst, so dass mit einer gewissen zeitlichen Verzögerung gleichfalls in und auf den Abschnitten unterschiedlich schnell nacheinander Farbumschläge infolge der Reaktion der Indikator-Verbindungen mit den in dem Harn als Exsudat befindlichen Substanzen auftreten in Abhängigkeit von der Exposition der Einlage gegenüber dem Harn.

In einem zusätzlichen Ausführungsbeispiel weist die Einlage eine Deckschicht mit drei Abschnitten auf, die sich von der Oberseite der Deckschicht bis zu deren Unterseite erstrecken. Gegen die Unterseite der Deckschicht ist eine Trägerschicht angesetzt und flächig auf der Deckschicht aufliegend. Der erste Abschnitt umfasst einen Vliesstoff mit verwirbelten Filamenten, der zweite Abschnitt hat einen Vliesstoff mit verwirbelten und glatten Filamenten und der dritte Abschnitt weist einen Vliesstoff mit glatten Filamenten auf. In den Vliesstoffen befindet Helianthin als Indikator-Verbindung, in anderen Ausführungsbeispielen Bromphenolblau, Kaliumpermanganat, Phenolphthalein oder Lackmus. Auf der Oberseite der Deckschicht ist eine die Polyvinylalkohol-Polymerverbindung enthaltende Außenschicht angeordnet. An zwei gegenüberliegenden Seiten der Einlage, nämlich an der unter der Deckschicht angeordneten Trägerschicht der Einlage, sind Seitenabschnitte mit Gewebe als textiles Flächengebilde angebracht sind; diese um die mit den Seiten übereinstimmenden Faltlinien in Richtung zu der Unterseite des Schrittbereichs des Schlüpfers gefaltet und in ihrem Faltzustand sind die Teile der Seitenabschnitte, die aufeinander liegend sind, miteinander mittels eines Heftstichs oder eines Wechselstichs vernäht. In einem anderen Ausführungsbeispiel sind die Teile der Seitenabschnitte, die aufeinander liegen, miteinander mittels einer die Marke eines Herstellers aufweisenden Papierlage verbunden. Aufgrund der unterschiedlichen Oberflächenvergrößerung der Vliesstoffe zeigt es bei diesen Ausführungsbeispielen sich, dass in und auf den Abschnitten unterschiedlich schnell Farbumschläge nacheinander infolge der Reaktion der Indikator-Verbindungen mit den in dem Harn als Exsudat befindlichen Substanzen auftreten in Abhängigkeit von der Exposition (Aussetzen) der Einlage gegenüber dem Exsudat. Der Farbumschlag in und auf dem ersten Abschnitt erfolgt bereits rasch nach einer Zeitdauer von ca. 15 min, der in und auf dem zweiten Abschnitt tritt nach ca. 30 min und der in und auf dem dritten Abschnitt tritt erst nach 3 Stunden auf.

In einem weiteren Ausführungsbeispiel weist die Einlage eine Deckschicht mit drei Abschnitten auf, die sich von der Oberseite der Deckschicht bis zu deren Unterseite erstrecken. Gegen die Unterseite der Deckschicht ist eine Trägerschicht angesetzt und flächig auf der Deckschicht aufliegend. Der erste Abschnitt umfasst einen Vliesstoff mit verwirbelten Filamenten, der zweite Abschnitt hat eine Gewebe oder einen Vliesstoff einhält, die Mikrokapseln als Indikator-Verbindung enthalten. Die Hüllen derselben sind mit einer Polyvinylalkohol-Polymerverbindung hergestellt. Infolge der Nutzung des Schlüpfers tritt auf dem ersten Abschnitt nach einer Zeitdauer von ca. 15 min ein Farbumschlag auf, hingegen ist ein solcher auf dem zweiten Abschnitt nach ca. 60 min zu beobachten.

### Vorteile der Erfindung

Durch das geschickte Zusammenwirken von Indikatormitteln und der elektronischer Sensoreinrichtung in der Einlage in der erfindungsgemäßen Vorrichtung erweist sich diese als besonders vorteilhaft zur Bestimmung der Dauer der Nutzung des Bekleidungsstücken und Schuhen durch

Vermeidung hoher volkswirtschaftlicher Schäden,
Nachweisbarkeit der Nutzung der Bekleidungsstücke und Schuhe und deren Dauer zweifelsfrei, eindeutig und ohne Weiteres ersichtlich,
zweifelsfreier bleibender Nachweis der Nutzung der Bekleidungsstücke und Schuhe und deren Dauer,
die Überprüfung des online-Handel bez. der Nutzung der Bekleidungsstücke und Schuhe und deren Dauer,
die Einschränkung missbräuchlicher dauerhafter Nutzung bereits durch das Bekanntsein des zweifelsfreien, eindeutigen Nachweisverfahrens,
die einwandfreie Unterscheidung zwischen dem einmaligen Anprobieren der Bekleidungsstücke und Schuhe einerseits und der -zeitlich darüber hinaus gehenden- andauernden Nutzung von Bekleidungsstücken und Schuhen andererseits,
die Beibehaltung des angenehmen Tragegefühls des Benutzers,
kein Auftreten die Verformbarkeit des Textils einschränkender Strukturen,
die preisgünstige Herstellung und einfache Anbringung in Bekleidungsstücken und Schuhen,
und
die leichte Entfernbarkeit nach dem Erwerb der Waren durch Benutzer,
die zuverlässig Anzeige der Dauer der Nutzung,
die einwandfreie überprüfbare Anzeige des mechanischen Eingriffs des Nutzers, der widerrechtlich oder unerwünscht ist, zwecks Entfernung der Einlage und
die Berücksichtigung der wirtschaftlichen Erfordernisse der kostengünstigen Herstellung der erfindungsgemäßen Vorrichtung
mit der Einlage als Massenware zur Benutzungskontrolle von Bekleidungsstücken und Schuhwerk.

## Patentansprüche

1. Vorrichtung zur Bestimmung einer durch einen Benutzer erfolgten Nutzung eines Bekleidungsstücks mit einer Einlage zur Anordnung auf einer dem Benutzer zugewandten Innenseite desselben und zur Aufnahme menschlicher Exsudate,
a. die Einlage Indikatormittel umfasst,
b. wobei die Indikatormittel mindestens eine Indikator-Verbindung aufweisen,
c. welche infolge ihrer Reaktion mit für menschliche Ausscheidungen charakteristischen Substanzen einen Farbumschlag zeigt,
d. die Einlage als ein textiles und/oder papierenes Flächengebilde ausgestaltet ist,
**dadurch gekennzeichnet, dass**
e. ein oberer Bereich der Einlage Abschnitte aufweist, die textile Flächengebilde unterschiedlicher Zusammensetzung und mindestens eine Indikator-Verbindung enthalten,
f. ein erster Abschnitt des oberen Bereichs der Einlage einen Vliesstoff mit verwirbelten Filamenten umfasst,
g. mindestens ein zweiter Abschnitt als textiles Flächengebilde einen Vliesstoff mit verwirbelten und glatten Filamenten umfasst.

2. Verwendung einer Vorrichtung zur Bestimmung einer durch einen Benutzer erfolgten Nutzung eines Bekleidungsstücks mit einer Einlage zur Anordnung auf einer dem Benutzer zugewandten Innenseite desselben und zur Aufnahme menschlicher Exsudate,
a. die Einlage Indikatormittel umfasst,
b. wobei die Indikatormittel mindestens eine Indikator-Verbindung aufweisen,
c. welche infolge ihrer Reaktion mit für menschliche Ausscheidungen charakteristischen Substanzen einen Farbumschlag zeigt,
d. die Einlage als ein textiles und/oder papierenes Flächengebilde ausgestaltet ist,
**dadurch gekennzeichnet, dass**
e. ein oberer Bereich der Einlage Abschnitte aufweist, die textile Flächengebilde unterschiedlicher Zusammensetzung und mindestens eine Indikator-Verbindung enthalten,
f. ein erster Abschnitt des oberen Bereichs der Einlage einen Vliesstoff mit verwirbelten Filamenten umfasst,
g. mindestens ein zweiter Abschnitt als textiles Flächengebilde einen Vliesstoff mit verwirbelten und glatten Filamenten umfasst.

3. Verwendung einer Vorrichtung zur Bestimmung der durch einen Benutzer erfolgten Nutzung eines Bekleidungsstücks nach Anspruch 2, **dadurch gekennzeichnet, dass** die Filamente mindestens eine kunststoffartige Polymer-Verbindung enthalten und als Indikator-Verbindungen Helianthin- (Methylorange), Bromphenolblau-, Kaliumpermanganat-, Phenolphthalein-, Lackmus- Verbindungen oder Mischungen derselben verwendet werden, die den pH-Wert der in dem oberen Bereich der Einlage aufgenommenen Ausscheidungen anzeigen.

4. Verwendung einer Vorrichtung zur Bestimmung der durch einen Benutzer erfolgten Nutzung eines Bekleidungsstücks nach Anspruch 2 oder 3, **dadurch gekennzeichnet, dass** die Indikator-Verbindung in Mikrokapseln eingeschlossen ist, deren Mäntel eine Polyvinylalkohol-Polymerverbindung enthalten, zur zumindest teilweisen Auflösung derselben in Gegenwart von Exsudat.

5. Verwendung einer Vorrichtung zur Bestimmung der durch einen Benutzer erfolgten Nutzung eines Bekleidungsstücks nach einem der Ansprüche 2 bis 4, **dadurch gekennzeichnet, dass** der obere Bereich eine Deckschicht ist, auf der Deckschicht eine die Polyvinylalkohol-Polymerverbindung enthaltende Außenschicht angeordnet ist, die in Gegenwart von Exsudat aufzulösen vermag.

6. Verwendung einer Vorrichtung zur Bestimmung der durch einen Benutzer erfolgten Nutzung eines Bekleidungsstücks nach einem der Ansprüche 2 bis 4, **dadurch gekennzeichnet, dass** die Einlage als oberen Bereich eine Deckschicht aufweist, die mindestens zwei Abschnitte aufweist,
auf der Oberseite der Einlage eine Beschichtung angeordnet ist, die als Oberschicht auf der Deckschicht als Außenschicht aufliegt, die Beschichtung als eine wasserdampfdurchlässige flüssigkeitsundurchlässige Schicht ausgestaltet ist, so dass zwar Dämpfe die Beschichtung durchdringen und mit den Indikatormitteln der Einlage zu den Farbumschlag erzeugenden Reaktionsprodukten reagieren, aber die Reaktionsprodukte nicht durch die Beschichtung in Richtung zu der mit dem Körper des Benutzers in Kontakt gelangbaren Oberseite dringen.

7. Verwendung einer Vorrichtung zur Bestimmung der durch einen Benutzer erfolgten Nutzung eines Bekleidungsstücks nach einem der Ansprüche 2 bis 6, **dadurch gekennzeichnet, dass** die Einlage mit dem Bekleidungsstück mittels eines Heftstichs oder eines Wechselstichs vernäht wird.

8. Verwendung einer Vorrichtung zur Bestimmung der durch einen Benutzer erfolgten Nutzung eines Bekleidungsstücks nach einem der Ansprüche 2 bis 7, **dadurch gekennzeichnet, dass** an zwei gegenüberliegenden Seiten der Einlage, insbesondere an einer unter der Deckschicht angeordneten Trägerschicht der Einlage, Seitenabschnitte angebracht sind, die um mit den Seiten übereinstimmende Faltlinien schwenkbar sind, in einem Faltzustand Teile der Seitenabschnitte aufeinander anordbar und miteinander verklebbar und/oder mittels einer Papierlage verbindbar sind.

9. Verwendung einer Vorrichtung zur Bestimmung der durch einen Benutzer erfolgten Nutzung eines Bekleidungsstücks nach einem der Ansprüche 2 bis 8, **dadurch gekennzeichnet, dass** die textilen und papierenen Flächengebilde eine wasseranziehende Polyethylenglykol-Verbindung und / oder hydrophile Filamente aufweisen, die als Kern-Mantel-Filamente ausgebildet sind und die Indikator-Verbindung aufweisen.

10. Verwendung einer Vorrichtung zur Bestimmung der durch einen Benutzer erfolgten Nutzung eines Bekleidungsstücks nach einem der Ansprüche 2 bis 9, **dadurch gekennzeichnet, dass**
die Einlage eine elektronische Sensor-Einrichtung aufnimmt, die einen die Dauer der Nutzung des Bekleidungsstücks über mindestens eine für die Dauer charakteristische Messgröße erfassenden Sensor zur Bestimmung der Abnutzung des Bekleidungsstücks umfasst,
die elektronische Sensoreinrichtung einen Sensor mit einer Empfindlichkeit für für menschliche Ausscheidungen charakteristische Substanzen aufweist,
der Sensoreinrichtung von dem Sensor ausgebbaren und/oder erfassten Messgrößen korrespondierende oder korrelierende Sensorsignale prozessierbar, wie detektierbar, speicherbar, anzeigbar und/oder ausgebbar macht.
die elektronische Sensoreinrichtung als elektronisches Bauelement ausgestaltet ist, welches die chemischen oder biochemischen oder physikalischen Messgrößen in der Einlage erfasst und an einen Empfänger weiterleitet,
der Empfänger das Sensorsignal als Ist-Wert mit in ihm eingegebenen Sollwerten vergleicht und nach Maßgabe der Sollwerte ein mit dem Sensorsignal korrespondierendes Ausgangssignal erzeugt und dieses an eine Speichereinrichtung weiter zur Speicherung und Ablesung leitet.

11. Verwendung einer Vorrichtung zur Bestimmung der durch einen Benutzer erfolgten Nutzung eines Bekleidungsstücks nach einem der Ansprüche 1 bis 10, **dadurch gekennzeichnet, dass** der obere Bereich der Einlage als eine Deckschicht ausgebildet ist, gegen eine Unterseite der Deckschicht eine Trägerschicht angesetzt ist, die Trägerschicht flüssigkeitsdicht ausgestaltet ist.

## Claims

1. A device for identifying a use of an item of clothing by a user, having a lining for arranging on an inner side of said item of clothing, facing the user, and for absorbing human exudate,
a. the lining comprises indicator means,
b. wherein the indicator means have at least one indicator compound,
c. which, as a consequence of its reaction with substances which are characteristic of human secretions, displays a colour change,
d the lining is configured as a textile flat structure and/or paper flat structure,
**characterised in that**
e. an upper region of the lining has sections which contain textile flat structures of differing composition and at least one indicator compound,
f. a first section of the upper region of the lining comprises a non-woven fabric with twisted filaments,
g. at least a second section comprises a non-woven fabric, with twisted and smooth filaments, as a textile flat structure.

2. Use of a device for identifying a use of an item of clothing by a user, having a lining for arranging on an inner side of said item of clothing, facing the user, and for absorbing human exudate,
a. the lining comprises indicator means,
b. wherein the indicator means have at least one indicator compound,
c. which, as a consequence of its reaction with substances which are characteristic of human secretions, displays a colour change,
d. the lining is configured as a textile flat structure and/or paper flat structure, **characterised in that**
e. an upper region of the lining has sections which contain textile flat structures of differing composition and at least one indicator compound,
f. a first section of the upper region of the lining comprises a non-woven fabric with twisted filaments,
g. at least a second section comprises a non-woven fabric, with twisted and smooth filaments, as a textile flat structure.

3. Use of a device for identifying the use of an item of clothing by a user according to Claim 2, **characterised in that** the filaments contain at least one plastics-like polymer compound and helianthin (methyl orange) compounds, bromophenol blue compounds, potassium permanganate compounds, phenolphthalein compounds, litmus compounds or mixtures thereof are used as indicator compounds, which indicate the pH value of the secretions absorbed in the upper region of the lining.

4. Use of a device for identifying the use of an item of clothing by a user according to Claim 2 or 3, **characterised in that** the indicator compound is enclosed in microcapsules, the shells of which contain a polyvinyl alcohol polymer compound, for at least partially dissolving them in the presence of exudate.

5. Use of a device for identifying the use of an item of clothing by a user according to any one of Claims 2 to 4, **characterised in that** the upper region is a top layer, and an outer layer containing the polyvinyl alcohol polymer compound is arranged on the top layer, this outer layer being able to dissolve in the presence of exudate.

6. Use of a device for identifying the use of an item of clothing by a user according to any one of Claims 2 to 4, **characterised in that** the lining has a top layer as an upper region, which top layer has at least two sections,
a coating is arranged on the upper side of the lining, which rests as an upper layer on the top layer as an outer layer, the coating is configured as a water-vapour-permeable, liquid-impermeable layer, so that vapour can penetrate the coating and react with the indicator means of the lining to give reaction products which produce the colour change, but the reaction products do not penetrate the coating toward the upper side, which can come into contact with the body of the user.

7. Use of a device for identifying the use of an item of clothing by a user according to any one of Claims 2 to 6, **characterised in that** the lining is sewn to the item of clothing by means of a tacking stitch or a cable stitch.

8. Use of a device for identifying the use of an item of clothing by a user according to any one of Claims 2 to 7, **characterised in that**, on two opposite sides of the lining, in particular on a carrier layer of the lining which is arranged under the top layer, lateral sections are attached which can be pivoted about fold lines which match the sides, in a folded state parts of the lateral sections can be arranged on one another and can be adhesively bonded to one another and/or can be joined to one another by means of a paper layer.

9. Use of a device for identifying the use of an item of clothing by a user according to any one of Claims 2 to 8, **characterised in that** the textile and paper flat structures have a water-attracting polyethylene glycol compound and/or hydrophilic filaments, which are formed as core-shell filaments and have the indicator compound.

10. Use of a device for identifying the use of an item of clothing by a user according to any one of Claims 2 to 9, **characterised in that**
the lining accommodates an electronic sensor apparatus, which comprises a sensor, which detects the duration of use of the item of clothing via at least one measurement variable which is characteristic of duration, for identifying the deterioration of the item of clothing,
the electronic sensor apparatus has a sensor with a sensitivity for substances which are characteristic of human secretions,
the sensor apparatus makes sensor signals corresponding to or correlating with measurement variables which are output and/or detected by the sensor processable, such as detectable, storable, displayable and/or outputable,
the electronic sensor apparatus is configured as an electronic component, which detects the chemical or biochemical or physical measurement variables in the lining and forwards them to a receiver,
the receiver compares the sensor signal as an actual value to setpoint values which have been input into it and, in accordance with the setpoint values, produces an output signal corresponding to the sensor signal and forwards this to a storage apparatus for storage and reading.

11. Use of a device for identifying the use of an item of clothing by a user according to any one of Claims 2 to 10, **characterised in that** the upper region of the lining is formed as a top layer, and a carrier layer is placed against an underside of the top layer, this carrier layer being configured in a liquid-tight manner.

## Revendications

1. Dispositif de détermination d'un mode d'utilisation par un utilisateur d'un vêtement avec un insert destiné à être disposé sur un côté interne de celui-ci, orienté vers l'utilisateur et destiné à l'absorption des exsudats humains,
a. l'insert comprend des moyens indicateurs,
b. dans lequel les moyens indicateurs comprennent au moins un composé indicateur,
c. qui, du fait de sa réaction avec des substances caractéristiques des sécrétions humaines, présente un changement de couleur,
d. l'insert est conçu comme une structure plane en textile et/ou papier,
**caractérisé en ce que**
e. une partie supérieure de l'insert comprend des portions qui contiennent des structures planes textiles de différentes compositions et au moins un composé indicateur,
f. une première portion de la partie supérieure de l'insert comprend un tissu non tissé avec des filaments entrelacés,
g. au moins une deuxième portion sous la forme d'une structure plane textile comprend un tissu non tissé avec des filaments entrelacéss et lisses,

2. Utilisation d'un dispositif de détermination d'un mode d'utilisation par un utilisateur d'un vêtement avec un insert destiné à être disposé sur un côté interne de celui-ci, orienté vers l'utilisateur et pour l'absorption des exsudats humains,
a. l'insert comprend des moyens indicateurs,
b. dans lequel les moyens indicateurs comprennent au moins un composé indicateur,
c. qui, du fait de sa réaction avec des substances caractéristiques des sécrétions humaines, présente un changement de couleur,
d. l'insert est conçu comme une structure plane en textile et/ou papier,
**caractérisée en ce que**
e. une partie supérieure de l'insert comprend des portions qui contiennent des structures planes textiles de différentes compositions et au moins un composé indicateur,
f. une première portion de la partie supérieure de l'insert comprend un tissu non tissé avec des filaments entrelacés,
g. au moins une deuxième portion sous la forme d'une structure plane textile comprend un tissu non tissé avec des filaments entrelacés et lisses.

3. Utilisation d'un dispositif de détermination d'un mode d'utilisation par un utilisateur d'un vêtement selon la revendication 2, **caractérisée en ce que** les filaments contiennent au moins un composé polymère de type matière plastique et 1"hélianthine (méthylorange), le bleu de bromophénol, le permanganate de potassium, le phénolphtaléine, le papier de tournesol ou des mélanges de ceux-ci sont utilisés en tant comme composés indicateurs , lesquels indiquent le pH des sécrétions logées dans la partie supérieure de l'insert.

4. Utilisation d'un dispositif de détermination d'un mode d'utilisation par un utilisateur d'un vêtement selon la revendication 2 ou 3, **caractérisée en ce que** le composé indicateur est inclus dans des microcapsules dont les enveloppes contiennent un composé polymère de type alcool polyvinylique, pour la dissolution au moins partielle de celles-ci en présence d'un exsudat.

5. Utilisation d'un dispositif de détermination d'un mode d'utilisation par un utilisateur d'un vêtement selon l'une des revendications 2 à 4, **caractérisée en ce que** la partie supérieure est une couche de revêtement et sur la couche de revêtement est disposée une couche externe contenant le composé polymère de type alcool polyvinylique, qui tend à se dissoudre en présence d'exsudat.

6. Utilisation d'un dispositif de détermination d'un mode d'utilisation par un utilisateur d'un vêtement selon l'une des revendications 2 à 4, **caractérisée en ce que**
l'insert comporte en tant que partie supérieure une couche de recouvrement qui comporte au moins deux portions,
un revêtement est disposé sur le côté supérieur de l'insert,
et repose en tant que couche supérieure sur la couche de recouvrement en tant que couche externe,
le revêtement est conçu en tant que couche perméable à la vapeur d'eau et imperméable aux liquides de sorte que bien que des vapeurs pénètrent dans le revêtement et réagissent avec les moyens indicateurs de l'insert aux produits de réaction produisant le changement de couleur, les produits de réaction ne pénètrent pas à travers le revêtement en direction du côté supérieur pouvant entrer en contact avec le corps de l'utilisateur.

7. Utilisation d'un dispositif de détermination d'un mode d'utilisation par un utilisateur d'un vêtement selon l'une des revendications 2 à 6, **caractérisée en ce que** l'insert est cousu avec le vêtement au moyen d'un point de faufilage ou d'un point alterné.

8. Utilisation d'un dispositif de détermination d'un mode d'utilisation par un utilisateur d'un vêtement selon l'une des revendications 2 à 7, **caractérisée en ce que**, sur deux côtés opposés de l'insert, plus particulièrement sur une couche de support de l'insert, disposée sous la couche de revêtement, sont appliquées des portions latérales qui peuvent pivoter autour de lignes de pliage coïncidant avec les côtés, , des parties des portions latérales pouvant, dans un état de pliage, être disposées les unes sur les autres et collées les unes avec les autres et/ou reliées au moyen d'une couche de papier.

9. Utilisation d'un dispositif de détermination d'un mode d'utilisation par un utilisateur d'un vêtement selon l'une des revendications 2 à 8, **caractérisée en ce que** les structures planes en textile ou en papier comprennent un composé de polyéthylène-glycol hygroscopique et/ou des filaments hydrophiles, qui sont conçus comme des filaments à âme et gaine, et le composé indicateur.

10. Utilisation d'un dispositif de détermination d'un mode d'utilisation par un utilisateur d'un vêtement selon l'une des revendications 2 à 9, **caractérisée en ce que**
l'insert contient un dispositif de capteur électronique qui comprend un capteur mesurant la durée d'utilisation du vêtement par l'intermédiaire d'au moins une grandeur de mesure caractéristique de la durée, pour la détermination de l'usure du vêtement,
le dispositif de capteur électronique comprend un capteur avec une sensibilité pour des substances caractéristiques des sécrétions humaines,
le dispositif de capteur électronique rend traitables, ainsi que détectables, enregistrables, affichables et/ou émettables des signaux de capteurs pouvant être émis par le capteur et/ou correspondant ou corrélés avec les grandeurs de mesure relevées,
le dispositif de capteur électronique est conçu comme un composant électronique qui mesure des grandeurs de mesure chimiques ou biochimiques ou physiques dans l'insert et les transmet à un récepteur,
le récepteur compare le signal du capteur, en tant que valeur effective, avec des valeurs de consigne entrées dans celui-ci et, selon les valeurs de consigne, génère un signal de sortie correspondant au signal du capteur et transmet celui-ci à un dispositif d'enregistrement pour l'enregistrement et la lecture.

11. Utilisation d'un dispositif de détermination d'un mode d'utilisation par un utilisateur d'un vêtement selon l'une des revendications 2 à 10, **caractérisée en ce que** la partie supérieure de l'insert est conçue comme une couche de revêtement, , une couche de support étant appliquée contre un côté inférieur de la couche de revêtement, la couche de support étant conçue de façon à être étanche aux liquides.
